# FASCICULE DE BREVET EUROPEEN

(11) **EP 1 119 760 B1**
(45) Date de publication et mention de la délivrance du brevet: **12.12.2007**
(21) Numéro de dépôt: 99970161.8
(22) Date de dépôt: 01.10.1999
(51) Int. Cl.: G01N 3/56, G01M 7/04, G01N 33/32

(54) **DISPOSITIF ET METHODE DE MESURE D'UN INDICE DE RESISTANCE A L'ENDOMMAGEMENT DE REVETEMENTS D'EMBALLAGES METALLIQUES**
VORRICHTUNG UND VERFAHREN ZUR MESSUNG EINES BESCHÄDIGUNGSWIDERSTANDIGKEITSINDEX FÜR BESCHICHTUNGEN VON METALLVERPACKUNGEN
DEVICE AND METHOD FOR MEASURING AN INDEX OF RESISTANCE TO DAMAGE OF METAL PACKAGE COATINGS

(30) Priorité: 06.10.1998 FR 9812639
(43) Date de publication de la demande: 01.08.2001
(73) Titulaire: ALCAN RHENALU, 75116 Paris (FR)
(72) Inventeur: NEEL, Olivier, F-38500 Voiron (FR); LOPEZ, Sébastien, F-38340 Voreppe (FR); SCHMIDT, Martin, Peter, F-38140 La Murette (FR); CHENAL, Bruno, F-38960 Saint-Etienne-de-Crossey (FR)
(74) Mandataire: Marsolais, Richard
(86) Numéro de dépôt international: PCT/FR1999/002336
(87) Numéro de publication internationale: WO 2000/020836

(56) Documents cités:
- EP-A- 0 588 157
- US-A- 4 507 953
- US-A- 4 608 854
- US-A- 4 958 511
- US-A- 5 531 095
- A.J.PERRY: "scratch adhesion testing of hard coatings" THIN SOLID FILMS, vol. 107, 1983, pages 167-180, XP002106728 cité dans la demande

## Description

### DOMAINE DE L'INVENTION

L'invention concerne le domaine de la caractérisation physico-chimique des revêtements d'emballages métalliques, typiquement des vernis, et plus particulièrement la résistance à l'endommagement par chocs tout au long du circuit de production, depuis la fabrication de l'emballage, le conditionnement du produit emballé, le stockage, le transport et la distribution jusqu'au consommateur final.

### ETAT DE LA TECHNIQUE

On connaît de nombreuses méthodes de caractérisation ou de mesure des propriété physico-chimique, y compris mécaniques, des revêtements d'emballages métalliques. Parmi toutes ces méthodes, certaines pourraient a priori être utilisables pour prévoir la résistance à l'endommagement du revêtement d'un emballage donné.

Ainsi, on sait mesurer la dureté ou la rayabilité d'un revêtement ou d'un vernis en utilisant une série de crayons à dureté croissante (de 2B, B, HB, F, H, 2H, 3H, etc..) et en notant la référence du crayon dont la pointe raye ledit revêtement (norme ASTM D 3363).

De même, on sait mesurer la résistance à l'abrasion à l'aide de l'abrasimètre Taber ® dont le principe consiste à appliquer sur un échantillon plan à tester et pendant un temps déterminé, des meules tournantes et à mesurer la perte de poids de l'échantillon résultant de l'abrasion (norme ASTM D 4060).

On connaît aussi la technique du micro-abrasimètre (micro-scratch tester en anglais) qui consiste à appliquer sur un échantillon plan un stylet sphérique, généralement un diamant avec une pointe de rayon de 200 µm. Le stylet est déplacé à une vitesse constante prédéterminée et avec une force d'appui croissante durant tout le déplacement, de sorte qu'un sillon de plus en plus profond se forme dans ledit revêtement jusqu'à son arrachage avec mise à nu du métal sous-jacent lorsque la force d'appui atteint une certaine valeur, qui peut donc servir à caractériser ledit revêtement.

La technique du micro-abrasimètre peut être mise en oeuvre par exemple à l'aide d'un micro-abrasimètre "MST-CSEMEX" du Centre Suisse d'Electronique et de Microélectronique de Neuchatel.

Cette technique est décrite par exemple par A.J. PERRY, " Scratch adhesion testing of hard coatings ", dans " Thin solids films 107 (1983), p.167-180.

Par ailleurs, le brevet US4507953 (Vandermersschee) d'avril 1985 décrit un test d'abrasion par une plateforme vibrante pour plusieurs échantillons, n'autorisant pas une évaluation individuelle ni surtout quantifiée.

### PROBLEMES POSES

D'une part, le fabricant d'emballages est confronté à une demande grandissante d'emballages nouveaux, que ce soit par leur forme, ou par leur aspect, c'est à dire par le décor.

Ainsi la durée de vie commerciale des emballages tend à diminuer, alors que par ailleurs le niveau de l'exigence de qualité technique tend à augmenter, ce qui exige du fabricant d'emballage une réactivité toujours plus rapide et une maîtrise ou un contrôle des processus de fabrication toujours plus grands.

D'autre part, compte tenu de cette nécessaire réactivité, le fabricant d'emballages doit disposer de moyens pour mettre en oeuvre cette réactivité. En particulier, dans le cas d'essais de revêtements intérieurs ou extérieurs, il importe de disposer de moyens de laboratoire pour tester rapidement toute idée nouvelle, et cela dans un délai très court et à un coût faible.

Ces considérations peuvent être illustrées par un problème particulier qui s'est posé à la demanderesse, celui de la mise au point d'un couvercle coloré à ouverture facile, en aluminium, pour boîte boisson.

Les couvercles de boîtes boisson sont typiquement incolores, étant revêtus seulement d'un vernis extérieur époxy-phénolique à 4 m²/g qui est pratiquement incolore.

Face à une demande de clients pour des couvercles colorés, une bande aluminium standard, mais revêtue sur sa face externe d'un vernis standard coloré, a été fabriquée ainsi que les couvercles correspondants sur une ligne standard de fabrication de couvercles. Cette ligne comprend d'abord une presse formant les ébauches de couvercles, puis une presse de finition, en ligne, formant l'incision, le rivet, posant l'anneau conduisant au couvercle fini.

On a observé que beaucoup de couvercles colorés présentaient un endommagement ressemblant à un " écaillage " (scuffing en anglais) selon une couronne du bord à sertir appelé aussi crochet de sertissage, avec mise à nu localisée du métal.

L'analyse du problème a montré que cette couronne correspondait en fait à la zone de contact d'un couvercle donné sur le bord à sertir du couvercle suivant dans la pile de couvercles et que l'endommagement observé trouvait sans doute son origine dans les nombreux petits chocs subis par chaque couvercle au contact des couvercles voisins dans la pile de couvercles, lors des transferts ou convoyages en continu sur les lignes de fabrication de couvercles, notamment entre la presse d'ébauchage et celle de finition.

La demanderesse a observé que ce phénomène existait déjà sur les couvercles standards incolores dont le vernis extérieur était aussi en partie écaillé, mais que cela était en fait visuellement sans importance compte tenu de la transparence du vernis standard et de la réflexion de la lumière par le substrat d'aluminium, de sorte qu'à l'oeil nu, on ne pouvait pas distinguer entre partie écaillée et partie non écaillée; et de ce fait, le défaut n'entraînait pas de rebut industriel.

La demanderesse a reconnu très vite qu'il n'était pas possible de poursuivre la mise au point d'un couvercle coloré en procédant de cette manière, c'est-à-dire simplement en incorporant des matières colorantes aux vernis utilisés habituellement, la fabrication de bande métallique spécifique et celle des couvercles correspondants sur ligne de production étant beaucoup trop longues et trop coûteuses, notamment par l'effet de la perturbation de la production standard.

Elle a donc recherché une méthode pour limiter au maximum les essais sur ligne de fabrication, et elle a notamment essayé d'abord de faire le bilan des différents essais de couvercles ou des différents couvercles standards fabriqués sur ligne industrielle, en ce qui concerne une possible relation ou corrélation entre les caractéristiques des différents revêtements, notamment une caractéristique physico-chimique du revêtement, et la résistance à l'endommagement.

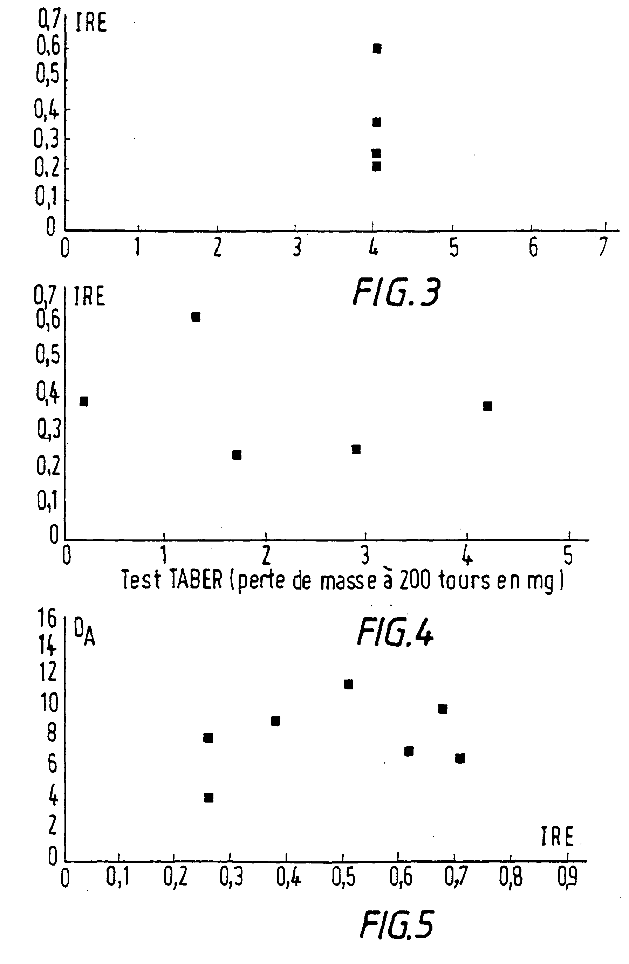

A cette fin, elle a testé les trois méthodes de caractérisation bien connues de l'homme du métier et mentionnées dans l'état de la technique. Aucune n'a conduit à une corrélation significative entre une propriété quelconque mesurée au laboratoire sur un échantillon d'emballage ou de métal ayant servi à fabriquer cet emballage, et la tenue à l'endommagement, comme illustré aux figures 3 à 5.

La demanderesse a donc recherché à résoudre ce problème et à mettre au point une méthode discriminante et générale qui permette d'évaluer rapidement, à un faible coût et sur un nombre limité d'échantillons, la résistance à l'endommagement " physique " de tous types de revêtement, typiquement par des chocs.

Un premier objet de l'invention est un dispositif et une méthode qui permettent de reproduire en laboratoire et très rapidement le type d'endommagement observé sur ligne de fabrication et de définir un indice de résistance à l'endommagement des revêtements. Un deuxième objet est l'application du premier objet au cas particulier des couvercles colorés.

### DESCRIPTION DE L'INVENTION

Le premier objet de l'invention est un dispositif de laboratoire pour caractériser la résistance à l'endommagement d'un revêtement protecteur, film plastique ou vernis, de tout ou partie d'un emballage, d'un substrat ou d'un élément d'emballage métallique comprenant une couche métallique, destiné à reproduire l'endommagement résultant de processus industriels donnés, typiquement dus à l'empilement ou au gerbage desdits emballages conduisant à des plages de contact, sous forme de pistes circulaires dans le cas d'emballages à bords circulaires, et permettant de mesurer un indice de résistance à l'endommagement IRE en abrégé - par un ratio de " zone non endommagée / zone totale considérée ", comprenant un moyen d'endommagement et caractérisé en ce que,
a) ledit moyen d'endommagement est soumis à un mouvement vibratoire assurant une succession de chocs, dans le sens vertical, contre ladite piste,
b) lors de chaque choc, ledit moyen ne s'applique simultanément que sur une fraction de ladite piste circulaire dudit emballage,
c) ledit moyen s'applique aléatoirement sur toute partie de ladite piste, par la libre rotation relative possible dudit moyen par rapport à ladite piste, d) l'action dudit moyen d'endommagement est réglable, typiquement en intensité, de manière à pouvoir notamment étalonner ledit dispositif par rapport audit processus industriel.

En pratique, il est commode de choisir un dispositif dans lequel ledit moyen et ladite piste sont, chacun, libres en rotation axiale.

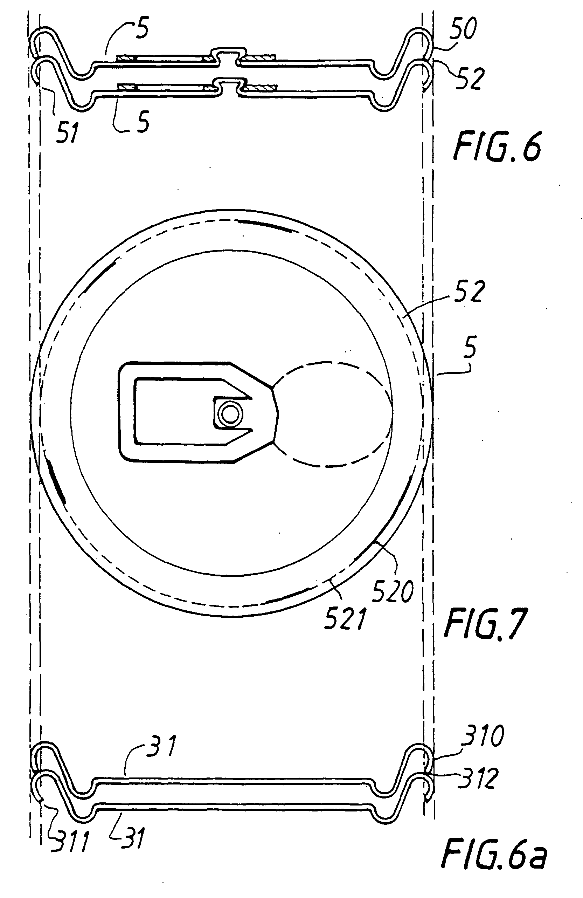

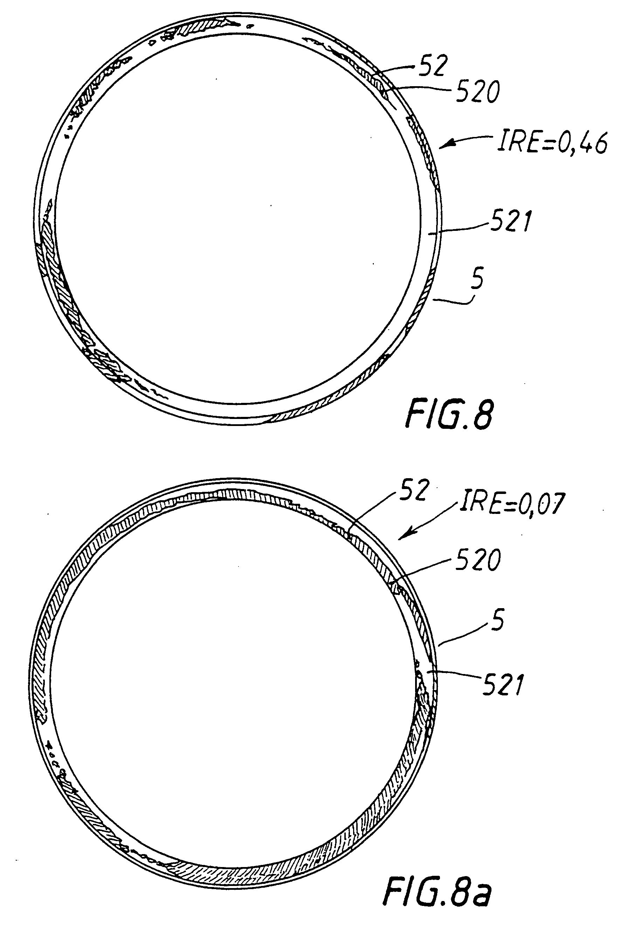

On entend par " piste circulaire " la bande relativement étroite (typiquement d'une largeur de quelques millimètres), fermée compte tenu de la symétrie axiale des emballages considérés, située dans un même plan horizontal, et sur laquelle apparaît l'écaillage du revêtement lors d'un processus industriel, comme illustré aux figures 7, 8 et 8a.

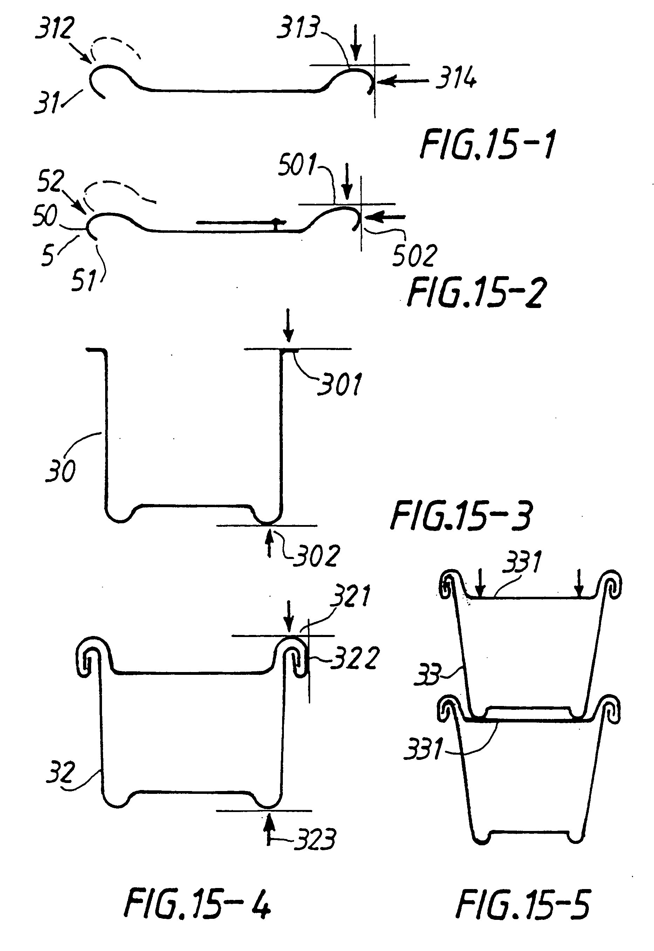

Cette piste peut aussi être légèrement tronconique, et se situer aux différents endroits les plus exposés d'un emballage où un endommagement est possible, comme illustré aux figures 15-1 à 15-5, par suite du frottement et des chocs entre les emballages eux-mêmes ou entre les emballages et les moyens de guidage ou de transfert de ces emballages.

D'une part, la demanderesse a eu la surprise de constater que ces moyens permettaient d'obtenir pratiquement exactement le même aspect d'endommagement, au point que, visuellement, il était difficile sinon impossible de distinguer un endommagement formé lors dudit processus industriel ou formé avec ledit dispositif.

D'autre part, la demanderesse a, comme cela sera détaillé ensuite, pu vérifier qu'effectivement, ledit dispositif reproduisait bien un endommagement du même type que celui formé lors dudit processus industriel, et était donc validé comme instrument de laboratoire.

Cette validation concerne à la fois le plan technique, dans la mesure où ledit dispositif donne des résultats reproductibles et prédictifs, le classement des revêtements par ordre d'IRE étant le même avec ledit dispositif et avec ledit processus industriel, et le plan économique, dans la mesure où une mesure de laboratoire est conduite sur un petit nombre d'emballages (à la limite un seul suffit), et ne dure typiquement que quelques minutes, alors qu'un essai sur ligne industrielle exige une grande quantité d'emballages à tester, prend des heures et peut coûter plusieurs milliers de francs français, notamment par les coûts des matières et de la main d'oeuvre impliqués aux différents stades de l'essai, sans compter la perte de production résultant de l'essai.

Avant d'arriver à cette définition des moyens qui permettent de résoudre le problème posé, la demanderesse avait étudié de nombreux dispositifs, notamment des dispositifs dans lesquels, en particulier, un des deux moyens mentionnés précédemment sous b) et sous c), ou leur combinaison, étaient absents.

En effet, si ces deux moyens sont absents, l'endommagement n'est plus semblable à celui obtenu lors dudit processus industriel, et le dispositif correspondant ne fournit plus une valeur d'endommagement corrélée avec celle obtenue à l'issue dudit processus industriel.

### DESCRIPTION DES FIGURES

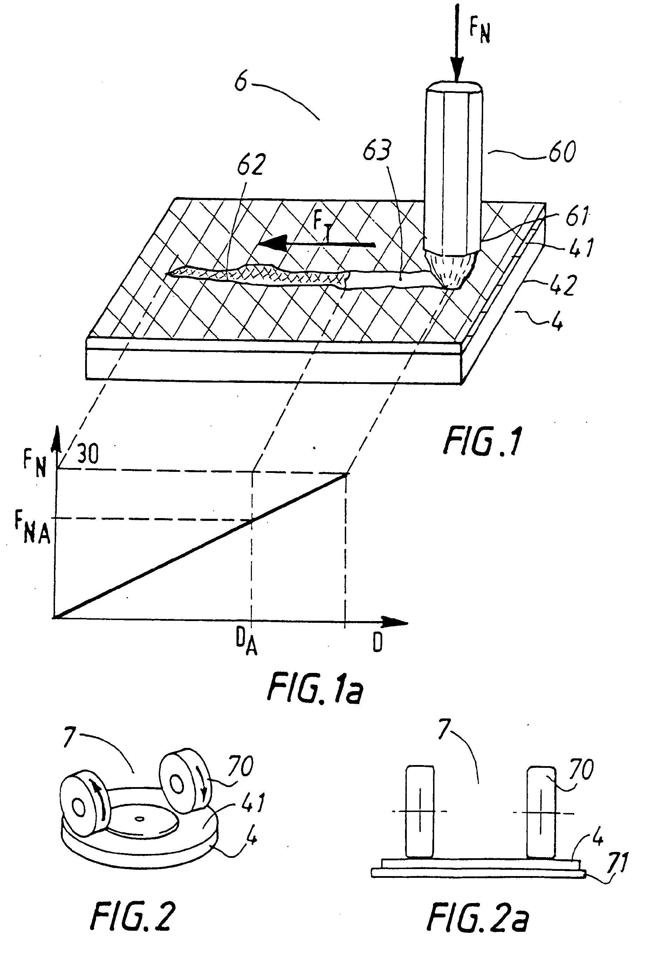

La figure 1 est une vue en perspective d'un micro-abrasimètre en cours de fonctionnement, qui explique le principe de la mesure : un stylet (60) à pointe de diamant (61) est appliqué avec une force verticale F_{N} sur un échantillon de bande à tester (4) comprenant une couche métallique (42) et un revêtement (41), typiquement une couche de vernis, ladite force allant croissant au fur et à mesure que le stylet se déplace à vitesse constante sur la distance D, comme représenté graphiquement sur le diagramme de la figure la.
   Sous l'action de la pointe (61) qui écrase de plus en plus le revêtement (41), une rayure (62) se forme, puis, pour une force critique F_{N} > F_{NA}, la rayure (63) présente le métal mis à nu. La distance D_{A} ou la force critique F_{NA} caractérisent donc ledit revêtement (41).
La figure 2 est une vue en perspective d'un abrasimètre Taber ® (7) et la figure 2a est une vue en coupe axiale. Dans ce dispositif, deux meules tournantes (70) sont appliquées avec une force prédéterminée sur le revêtement (41) d'une portion de bande métallique (4) portée par une sole tournante (71). On mesure la perte de poids (mg) par abrasion de l'échantillon après un nombre prédéterminé de tours, typiquement 200.
Les figures 3 à 5 illustrent l'absence de corrélation entre les valeurs d'IRE mesurées à l'issue dudit processus industriel pour différents revêtements testés, et les trois types de caractérisation initialement essayées par la demanderesse :
   La figure 3 porte en ordonnée les valeurs d'IRE et en abscisse la dureté du crayon, les chiffres de 1 à 7 ayant remplacé les lettres habituelles (1=2B, 2=B, 3=HB, 4=F, 5=H,...). La figure 4 illustre le test Taber ®. Elle porte en ordonnée les valeurs d'IRE et en abscisse la perte de poids en mg pour 200 tours de sole tournante.
   La figure 5 illustre le test de micro-rayure. Elle porte en ordonnée la distance D_{A} en mm, et en abscisse l'IRE
Les figures 6 et 7 sont relatives à des couvercles (5), alors que la figure 6a est relative à une ébauche de couvercle (31), les figures 6 et 6a étant des vues en coupe et la figure 7 une vue de dessus.
   A la figure 6, le couvercle (5) présente un rivet, un anneau, une incision (non représentée) et un crochet de sertissage (50) se terminant par sa tranche (51). Compte tenu de la courbure dudit crochet, cette tranche (51) prend appui sur le crochet du couvercle suivant selon une circonférence ou piste (52) plane ou légèrement tronconique, selon la pente dudit crochet au point d'impact de ladite tranche.
   De même, à la figure 6a, dans le cas d'une ébauche de couvercle (31), le crochet de sertissage (310) se termine par sa tranche (311), cette tranche prenant appui sur le crochet de l'ébauche suivante selon une circonférence de contact (312).
   Sur la figure 7 est représentée la circonférence de contact ou piste (52) obtenue à l'issue dudit processus industriel : elle comprend une succession d'arcs (520,521) correspondants à une alternance de zones soit où le métal est mis à nu (520), soit où le revêtement a résisté (521), l'IRE étant le rapport " somme des longueurs d'arcs (520) où le métal est mis à nu / périmètre de la circonférence de contact (52)".
Les figures 8 et 8a illustrent des circonférences de contact (52) avec portions endommagées (520) et portions sensiblement intactes (521), avec IRE=0,46 pour la figure 8 et IRE=0,07 pour la figure 8a.
   La détermination de l'IRE peut se faire aisément directement sur couvercles, du moins dans le cas de certains couvercles colorés, compte tenu de la grande différence de pouvoir réflecteur entre parties où le métal a été mis à nu et parties où subsiste un revêtement. Pour les vernis clairs ou transparents qui ne donnent pas un contraste suffisant, on peut révéler les zones de métal à nu à l'aide d'une solution acidifiée de sulfate de cuivre.

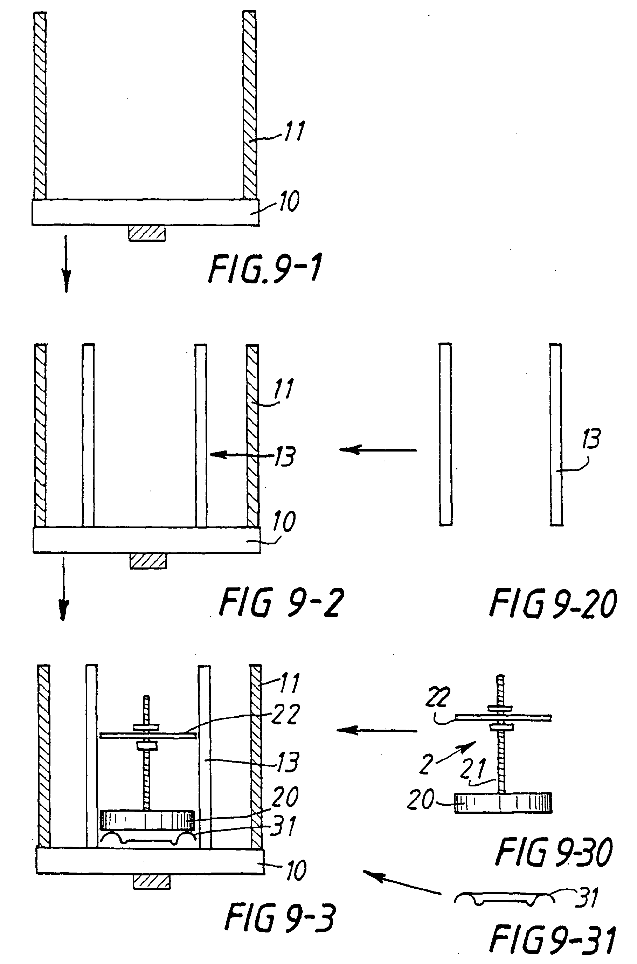

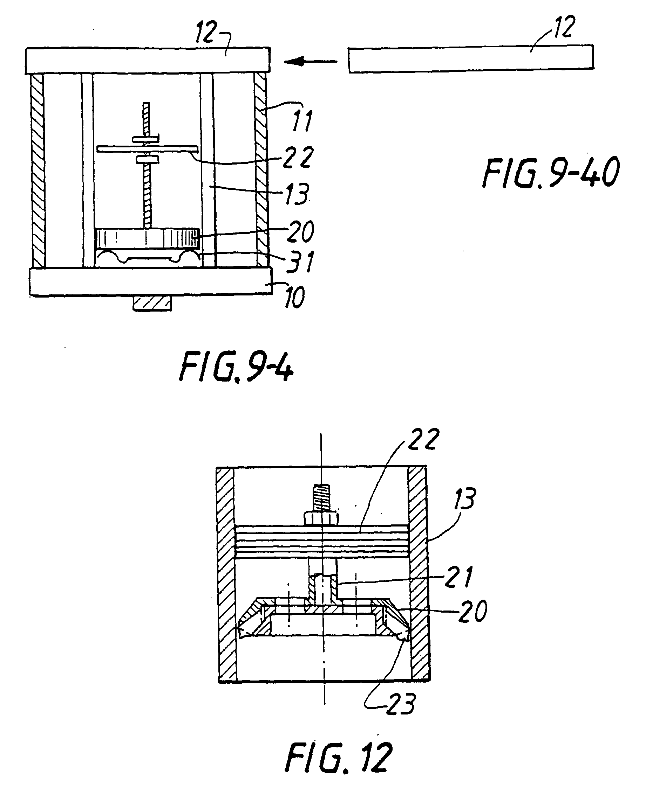

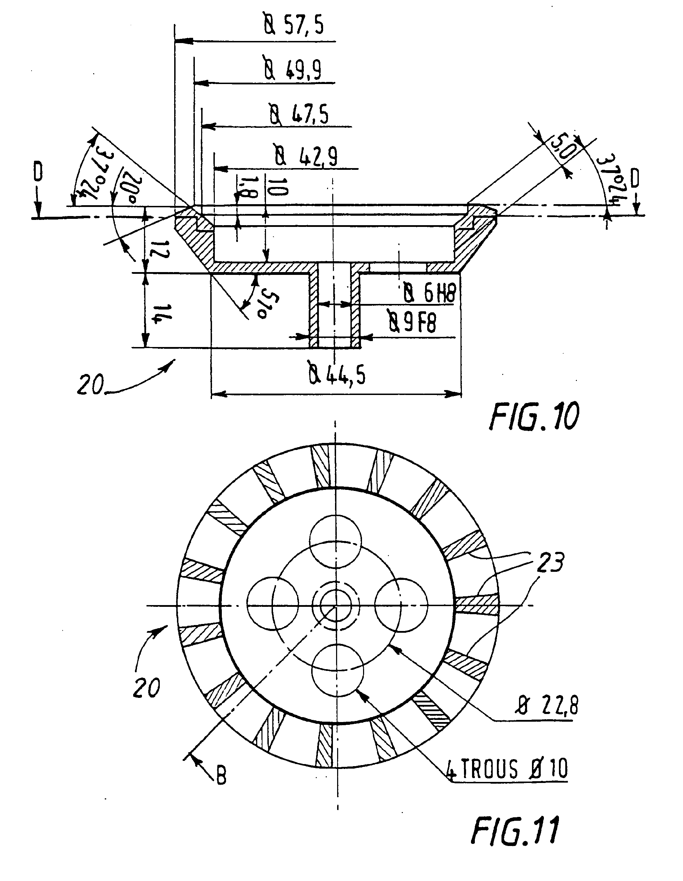

Les figures 9-1 à 9-40 et les figures 10 à 12 sont relatives au dispositif (1) selon l'invention. Toutes les figures sont des coupes axiales verticales, sauf la figure 11 qui est une coupe, dans le plan horizontal, selon l'axe A-A de la figure 10.

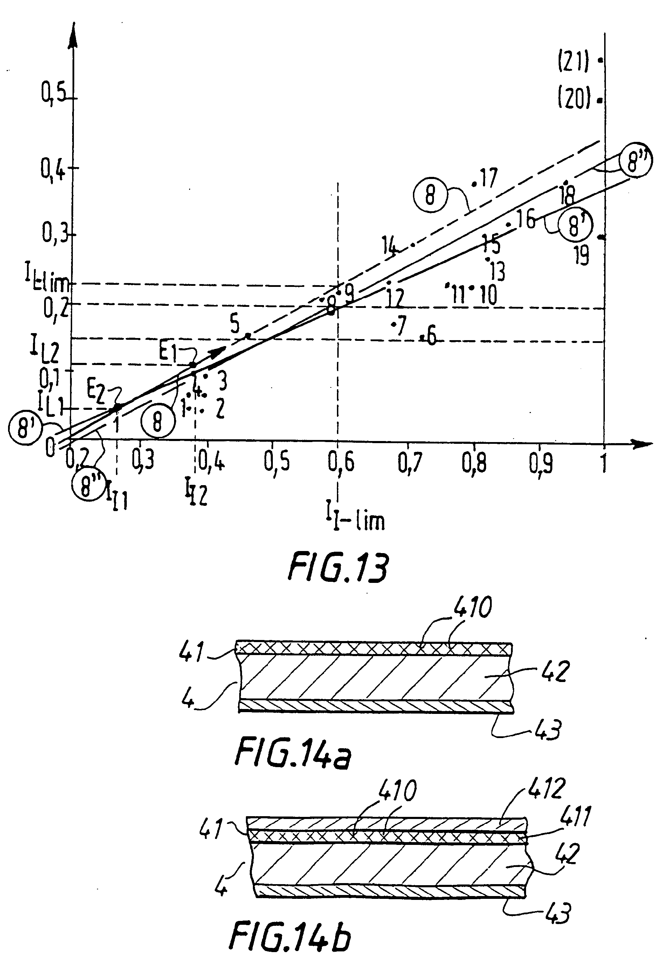

La figure 13 est un diagramme portant en abscisse les valeurs d'IRE I_{I} relatives à l'endommagement d'emballages Eᵢ sur ligne industrielle, et en ordonnée, les valeurs correspondantes I_{L} mesurées avec le dispositif et la méthode selon l'invention.
   La figure 13 porte un première droite (8) d'étalonnage ou de corrélation passant par les deux points de référence E₁ et E₂, une seconde droite (8') de corrélation portant sur ces deux points ainsi que les 6 essais référencés de 1, 1bis à 5, destinés à confirmer ou valider ladite première droite (8), et enfin une troisième droite (8") de corrélation portant sur l'ensemble des essais, y compris les essais de revêtements sélectionnés selon l'invention référencés de 6 à 19.
Les figures 14a et 14b représentent, en coupe, deux structures typiques de bande métallique (4) utilisée pour la fabrication des couvercles colorés selon l'invention qui comprennent une couche métallique centrale (42), typiquement en alliage d'aluminium, une couche de vernis intérieur (43), et, à l'extérieur, soit un revêtement monocouche de vernis (41) coloré par une charge pigmentaire (44) comme représenté à la figure 14a, soit un revêtement bicouche de vernis, avec une couche de vernis d'accrochage (410) colorée par une charge pigmentaire (44) et une couche externe de vernis (411), de préférence incolore, comme représenté à la figure 14b.
Les figures 15-1 à 15-5 sont des coupes de différents emballages ou d'éléments d'emballages illustrant d'autres localisations possibles d'endommagements de revêtements - localisations représentées par des flèches qui correspondent à des zones de contact naturelles qui courent le plus de risques d'endommagement lors desdits processus industriels, la présente invention pouvant être utilisée pour la mise au point de revêtements ne présentant pas de tels endommagements.
   La figure 15-1 représente une ébauche de couvercle (31) sur laquelle ont été indiqués trois localisations possibles : la localisation (312) due à l'empilage des couvercles, la localisation (313) correspondant à un contact ou frottement selon la verticale, la localisation (314) correspondant à contact ou frottement latéral.
   De même, à la figure 15-2, un couvercle (5) peut présenter des zones analogues avec les repères correspondants (52), (501) et (502).
   La figure 15-3 correspond à un corps de boite (30) sur lequel sont portées deux localisations, l'une sur le bord tombé (301), et l'autre sur la nervure (322) du fond de boîte.
   La figure 15-4 correspond à une boite sertie (32) sur laquelle ont été portées trois localisations, deux (321,322) sur les parties horizontales et verticales du couvercle, une sur la nervure (323) du fond de boîte.
   La figure 15-5 correspond à un gerbage de boîtes empilables où est portée la zone de contact (331) du fond d'une boîte en appui sur le couvercle de celle située au-dessous.

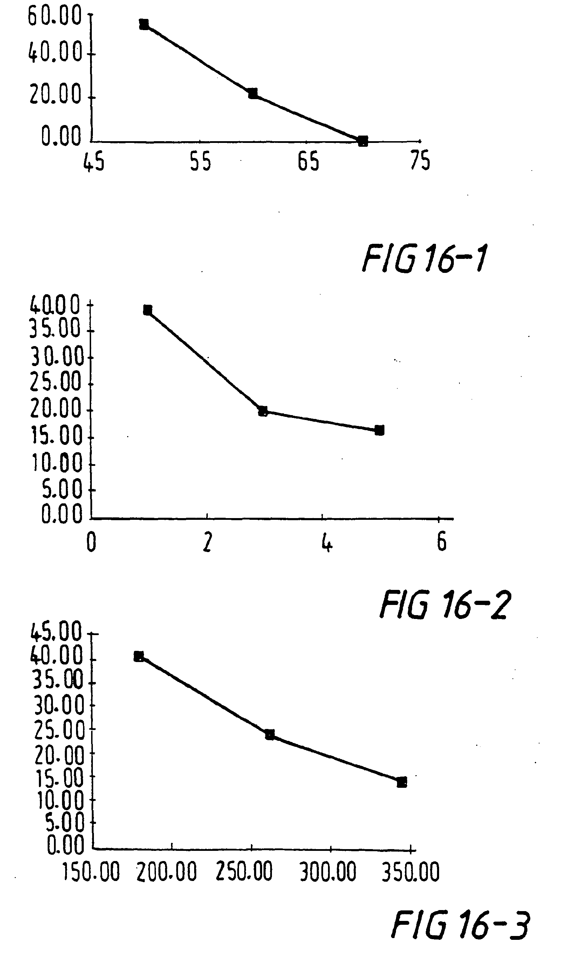

Les figures 16-1 à 16-3 sont des diagrammes illustrant l'influence sur l'IRE (en ordonnée) de différents paramètres opératoires du dispositif selon l'invention :
   - le réglage de la fréquence et/ou amplitude des vibrations, comme porté (en % d' " intensité " des vibrations - réglage de 0 à 100 de la table vibrante) en abscisse sur la figure 16-1,
   - la durée du test, comme portée (en min) en abscisse sur la figure 16-2,
   - la masse totale de l'outil d'endommagement en faisant varier le nombre de rondelles, comme porté (en g) en abscisse sur la figure 16-3, la masse de l'outil contribuant, avec le facteur fréquence et/ou amplitude des vibrations, à l' " intensité " de l'endommagement.

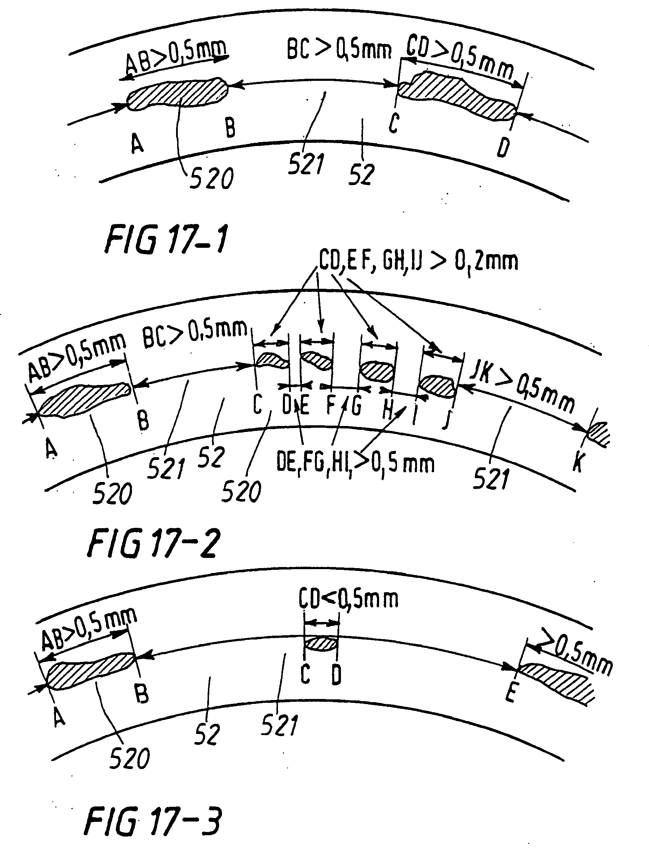

Les figures 17-1 à 17-3 illustrent la manière de mesurer l'IRE, en complément des figures 8 et 8a, en considérant différents types de portions endommagées (520) et de portions intactes (521) des pistes ou circonférences de contact (52).
   La figure 17-1 montre que l'on considère comme portions endommagée (520) toute portion de longueur supérieure à 0,5 mm (cas de AB et de CD), et comme portion non endommagée, toute portion BC supérieure à 0,5 mm.
   A contrario, comme illustré à la figure 17-2, des zones non endommagées de longueur inférieure à 0,5 mm sont considérées comme endommagés : la portion d'arc CJ est considérée comme une portion d'arc endommagé (520) - à condition toutefois que les zones endommagées aient une taille suffisante (> 0,2 mm).
   A contrario également, comme illustré à la figure 17-3, une portion endommagée isolée de moins de 0,5 mm de longueur (portion CD) ne sera pas comptabilisée comme portion d'arc endommagée. L'arc BE est ainsi considéré comme une portion non endommagée (521).

### DESCRIPTION DETAILLEE DE L'INVENTION

D'un point de vue pratique, le dispositif selon l'invention peut comprendre, comme représenté aux figures 9-1 à 12 :
a) une table vibrante (10), à taux de vibration ou efficacité modulable en fréquence et/ou amplitude, mais typiquement en amplitude,
b) un tube de guidage vertical (13) fixé à ladite table vibrante (10), dans lequel peut glisser ou tourner **autour d'un axe vertical**, sans frottement notable, ledit emballage ou élément d'emballage métallique, et, au-dessus de celui-ci, un outil d'endommagement (2),
c) ledit outil d'endommagement (2) comprenant une couronne (20) munie de dents (23) régulièrement espacées les unes des autres, de dureté élevée supérieure à celle de ladite couche métallique, typiquement en carbure de tungstène, surmontée d'une tige axiale (21) portant une masselotte (22) formée d'un empilement d'un nombre de rondelles d'acier (220) choisi en fonction de ladite intensité, ledit outil (2) étant placé dans ledit tube de guidage (13).

En effet, l'intensité de l'endommagement varie d'une part avec la masse dudit outil d'endommagement (voir figure 16-3), ledit nombre de rondelles d'acier constituant donc un des paramètres permettant de fixer des conditions opératoires discriminantes en ce qui concerne la mesure de la résistance à l'endommagement, et elle varie d'autre part avec le réglage propre de l'efficacité (réglage de la fréquence et/ou de l'amplitudes des vibrations) de la table vibrante (voir figure 16-1).

Un tel dispositif (1) est représenté aux figures 9-1 à 9-40 qui schématisent le montage dudit dispositif : deux colonnes (11) étant fixées au plateau de la table vibrante (10), on place successivement, le tube de guidage (13) que l'on centre sur ledit plateau, puis, dans le tube, l'ébauche de couvercle (31) à tester ainsi que l'outil d'endommagement (2), l'ébauche de couvercle (31) et l'outil (2) ayant un jeu typiquement de l'ordre de quelques mm (1 à 2 mm) pour que le tube ne freine pas la vibration axiale selon l'axe (9) vertical. On fixe ensuite la bride de maintien (12) aux colonnes de fixation (11) de manière à comprimer ledit tube (13) et à l'immobiliser par rapport audit plateau pour qu'il ne glisse pas latéralement durant toute la durée du dit mouvement vibratoire.

Un autre objet de l'invention est une méthode de mesure d'un IRE obtenu avec le dispositif (1) décrit précédemment, et corrélé à l'endommagement résultant d'un processus industriel, dans laquelle :
a) on approvisionne au moins un type d'emballage ou d'élément d'emballage donné Eₒ et on le soumet audit processus industriel, de manière à obtenir un emballage E_{I} dont le revêtement a subi un certain endommagement à l'issue dudit processus industriel, et sur lequel on mesure une valeur I_{I} pour l'IRE,
b) on soumet ledit emballage ou élément d'emballage Eₒ, comprenant ladite zone de contact, audit dispositif durant une durée et avec une dite intensité choisies de façon à obtenir un emballage modifié en laboratoire E_{L}, présentant un endommagement dont la valeur IL mesurée est typiquement voisine de 0.3.I_{I}, et par exemple comprise entre 0,2.I_{I} et 0,7.I_{I}, et ainsi à fixer, une fois pour toutes pour un processus industriel donné, des conditions opératoires dudit dispositif pour obtenir un niveau d'endommagement voisin de celui obtenu lors dudit processus industriel, ou de préférence supérieur, et ainsi de pouvoir évaluer la résistance à l'endommagement desdits revêtements (41) étudiés, classer les revêtements par IRE croissant et sélectionner au moindre coût et rapidement les revêtements les plus performants.

La méthode et le dispositif (1) selon l'invention ont été utilisés pour résoudre le problème exposé précédemment d'endommagement d'ébauches de couvercles colorés sur ligne de fabrication industrielle.

Les éléments particuliers à ce cas concret, qui ont permis d'étalonner ledit dispositif et de fixer les paramètres variables de ladite méthode sont les suivants :
- ledit emballage Eₒ est une ébauche (31) de couvercle rond de boîte boisson de 59,5 mm de diamètre extérieur, revêtu extérieurement d'un vernis époxy-phénolique à 4 g/m² de la Société HOLDEN, référence X1132 SLE.
- ledit dispositif (1) comprend :
   * une table vibrante (10) de marque RETSCH ®, type VIBRO ® fonctionnant à 50 Hz et à amplitude variable,
   * un tube de guidage (13) en PVC de 60 mm de hauteur, 60 mm de diamètre intérieur, de 6,2 mm d'épaisseur,
   * un outil d'endommagement (2) comprenant une couronne (20) munie de 15 dents (23) régulièrement espacées angulairement et de poids égal à 139 g, ladite fraction, égale à fraction de longueur de circonférence où se trouvent des dents, étant égale à 25%, une tige axiale (21) de 36 mm de hauteur portant une masselotte (22), constituée de n rondelles d'acier (220), chacune de poids unitaire égal à 41 g,
- on prend n égal à 3, le poids total dudit outil étant alors de 262g, on fixe ladite durée à 3 min, et on règle le taux de vibration ou d'efficacité de ladite table vibrante à 60%, ces paramètres opératoires étant fixés pour obtenir une valeur I_{L} de 0, 11, de l'ordre de 0,3.I_{I}, de manière à ce que l'endommagement avec le dispositif de laboratoire soit plus sévère que l'endommagement lors du processus industriel et que la transposition d'une sélection desdits revêtements (41), du laboratoire au processus industriel, soit d'autant plus fiable, ledit processus étant typiquement le convoyage des ébauches (31) de couvercles sur toute la ligne de production de couvercles qui conduit typiquement à une valeur I_{I} de 0,38.

Cette méthode n'utilise donc qu'un seul essai industriel de référence à partir d'un emballage donné. On peut également utiliser la méthode suivante, plus complète, qui fait appel à deux essais industriels de référence, à partir de deux emballages donnés.

Dans cette méthode, qui est une variante de la première :
a) on approvisionne deux types d'emballages E₁ et E₂, conduisant à l'issue dudit processus industriel, à des valeurs distinctes, et de préférence éloignées l'une de l'autre, I_{II} et I_{I2} pour l'IRE,
b) on soumet un des emballages, par exemple E₂, audit dispositif (1) de laboratoire et on règle ladite durée et intensité, de manière à obtenir une valeur d'endommagement correspondante I_{L2} telle que I_{L2} soit de l'ordre de 0,3. I_{I2} et, par exemple typiquement compris entre 0,2.I_{I2} et 0,7.I_{I2},
c) les réglages de durée et intensité étant déterminés et fixés en b), on soumet l'emballage E_{I} ou tout autre emballage à étudier, audit dispositif et on mesure la valeur I_{LI} correspondante,
d) à partir des deux couples de valeurs L_{LI} et I_{II}, I_{L2} et I_{I2}, on obtient un droite d'étalonnage et de corrélation (8) entre les valeurs d'endommagement des revêtements (41) mesurées sur emballages à l'issue d'un processus industriel et à l'issue de l'endommagement avec ledit dispositif (1), de façon à pouvoir, compte tenu d'une valeur limite I_{I-lim} reconnue comme acceptable pour ledit emballage à l'issue dudit processus industriel, calculer une valeur théorique correspondante I_{L-lim} ainsi qu'une valeur pratique compte tenu des intervalles de confiance associés à une seule mesure avec ledit dispositif, et sélectionner rapidement et au moindre coût lesdits revêtements (41) susceptibles de convenir à l'échelle industrielle.

Dans cette seconde méthode :
- lesdits emballages E₁ et E₂ sont des ébauches (31) de couvercles de boîte boisson, l'emballage E₁ étant revêtu extérieurement d'un vernis époxy-phénolique phénolique à 4 g/m² de la Société HOLDEN, référence X1132 SLE, l'emballage E₂ étant revêtu extérieurement d'un vernis époxy-phénolique phénolique à 4 g/m² de la Société HOLDEN, pigmenté orange de référence HE1236.
- les mêmes dits paramètres opératoires étant conservés, la valeur d'IRE I_{L2} relative à l'emballage E₂ est déterminée avec ledit dispositif et trouvée égale à 0,045,
- on mesure les valeurs correspondantes de I_{I1} (= 0,38) et I_{I2} (=0,26) sur lesdits emballages E₁ et E₂ issus dudit processus industriel,
- ladite valeur limite I_{I-lim} ayant été estimée à 0,60, on détermine, sur la base de ladite droite d'étalonnage ou de corrélation (8), ladite valeur limite théorique E_{L-lim} correspondante de 0,23 et une valeur pratique de 0,15 compte tenu des intervalles de confiance.

La figure 13 illustre, à titre d'exemple, cette seconde méthode de l'invention pour étalonner ledit dispositif (1) : les deux emballages à revêtements différents E₁ et E₂ sont soumis d'une part audit processus industriel pour donner des emballages " endommagés " avec des valeurs d'IRE de E_{I1} et E_{I2} portées sur l'axe des abscisses valant respectivement 0,38 pour I_{I1} et 0,26 pour I_{I2}, et ils sont d'autre part testés avec ledit dispositif (1) de laboratoire pour donner des emballages " endommagés " avec des valeurs d'IRE de E_{L1} et E_{L2} portées sur l'axe des ordonnées valant respectivement 0,11 pour I_{L1} et 0,045 pour I_{L2}. Lors de l'essai du premier produit E₁, les paramètres du procédé et dudit dispositif sont ajustés et fixés - une fois pour toutes pour un processus industriel donnée - de manière à obtenir une valeur I_{L1} d'IRE de 0,11, valeur choisie à environ 0,3 fois I₁₁, de manière à ce que les paramètres soient discriminants pour distinguer les meilleurs revêtements parmi les bons et non pour distinguer les moins mauvais parmi les pires.

Le second emballage E₂ ainsi que tous les échantillons relatifs à ce même processus industriel sont ensuite testés avec ces mêmes paramètres.

Compte tenu des exigences de qualité requises, il a été défini une valeur limite I_{I-lim} au-dessous de laquelle les couvercles issus dudit processus industriel présentent un endommagement inacceptable - du moins dans le cas des couvercles colorés - et ne peuvent être commercialisés. A cette valeur limite d'IRE, égale à 0,60, correspond, compte tenu de la droite de corrélation (8) passant par les deux points E₁ et E₂ du diagramme de la figure 13, une valeur limite théorique I_{L-lim} de 0,23 pour les mesures effectuées avec ledit dispositif (1).

Cette valeur est théorique dans la mesure où la pente de la droite de corrélation présente une large incertitude compte tenu du fait qu'elle est basée sur deux points seulement. Aussi, compte tenu des intervalles de confiance, et pour ne pas écarter, lors des mesures de laboratoire sur un seul essai, des produits éventuellement bons sur ligne industrielle, la valeur limite pratique I_{L-lim} pour tester un produit à l'échelle industrielle a été fixée à 0,15.

Les conditions d'essais conduisant à ces valeurs d'IRE sont indiquées notamment dans les exemples de réalisation.

Ainsi, en partant de deux emballages différents issus dudit processus industriel, c'est à dire deux types d'ébauches différant par ledit revêtement en ce qui concerne le critère d'endommagement, on peut établir une corrélation entre processus industriel et laboratoire pour tout emballage testé.

La présente méthode ne limite bien entendu pas le nombre d'essais industriels de référence: le fait d'augmenter ce nombre au-delà de deux n'entraîne pas de gain en matière de précision prédictive de la méthode, mais lui fait perdre un peu de sa rapidité et de sa simplicité qui fait partie de ses objectifs.

Selon un autre objet de l'invention, on peut remplacer, dans la méthode selon l'invention et en ce qui concerne l'utilisation dudit dispositif (1) de laboratoire, ledit emballage Eo, ou lesdits emballages E₁ et E₂ par les matériaux de départ correspondants, ledit moyen d'endommagement étant dès lors appliqué non pas sur un emballage, mais sur le revêtement (41) de ladite bande métallique (4) destinée à former ledit emballage métallique, l'échantillon à tester étant sous forme d'un disque plan, de façon à ce qu'il ait une libre rotation axiale durant la mise en oeuvre dudit dispositif.

Dans la méthode selon l'invention, les IRE sont mesurés soit par un rapport de longueur de piste ou couronne non endommagée. (521) sur la longueur totale de piste ou de couronne, soit par un rapport de surface non endommagée sur surface totale considérée, une partie endommagée étant une partie d'emballage où ladite couche métallique a été mise à nu suite audit endommagement, ladite longueur totale de piste ou de couronne correspondant typiquement au rebord circulaire d'un corps de boîte (30,32,33), d'un couvercle (5), ou d'une boîte sertie (32,33).

Une partie endommagée étant une partie d'emballage où ladite couche métallique a été mise à nu suite audit endommagement, ladite longueur totale de piste ou de couronne (52,312) correspondant typiquement au rebord circulaire d'un corps de boîte (30,32,33), d'un couvercle (5), ou d'une boîte sertie (32,33), les indices de résistance à l'endommagement (IRE) utilisés dans les méthodes précédentes sont mesurés de la façon suivante : sur le périmètre défini par la piste d'usure, on comptabilise les plages de vernis intactes.

Sont prises en compte dans les plages intactes les plages d'une longueur supérieure à 0,5 mm ne comportant aucun défaut de taille supérieure à 0,5 mm.

Sont considérés comme défauts de taille supérieure à 0,5 mm :
- les zones d'apparition continue de métal, de plus de 0,5 mm,
- les zones de plus de 0,5 mm constituées de zones d'apparition du métal dont la taille est comprise entre 0,2 et 0,5 mm et séparées par moins de 0,5 mm.

L'IRE est calculé en rapportant le cumul des longueurs de plages de vernis intact au périmètre total de la piste d'usure. On entend ici par longueur d'une plage sa longueur d'arc ou longueur curviligne.

Les figures 17-1 à 17-3 illustrent la manière retenue pour évaluer l'IRE.

La fraction de piste non endommagée (521) peut donc être évaluée manuellement par une mesure de longueur curviligne ou d'angle, comme cela est illustré aux figures 8 et 8a. Pour cette évaluation, d'autres méthodes pourraient être envisagées, typiquement par analyse d'images. Il importe seulement que la même " règle du jeu " s'applique en toutes circonstances. La règle retenue et indiquée ci-dessus conduit aux valeurs d'IRE de 0,46 et de 0,07 en ce qui concerne les figures 8 et 8a respectivement.

La méthode peut être avantageusement appliquée à des couvercles (5) comprenant un revêtement extérieur coloré ou non (41) caractérisés en ce qu'ils présentent un indice IL de résistance à l'endommagement IRE mesuré avec le dispositif de laboratoire dans les conditions précitées, d'au moins 0,15. Sur la base de tous les essais réalisés à la fois en laboratoire et sur ligne industrielle, comme représenté à la figure 13, on observe que pratiquement tous les produits présentant une valeur d'I_{L} supérieure à 0,15 présentent une valeur d'I_{I} supérieure à 0,6.

De préférence, lesdits couvercles (5) présentent un indice IL d'au moins 0,20, ou, pour les cas les plus exigeants, un indice IL supérieur à 0,25. Dans ce cas, la plupart des couvercles présentent une valeur d'I_{I} supérieure à 0,70.

Il est à noter que des couvercles non colorés avec un IRE élevé peuvent aussi être recherchés en particulier dans le cas où le revêtement ne doit pas être endommagé, indépendamment de toute considération d'aspect visuel.

Selon l'invention, ledit revêtement extérieur (41) peut comprendre une matière colorante à une teneur pondérale T (teneur pondérale sur revêtement ou vernis sec), sous forme soit d'une charge pigmentaire (410) de 0,5 à 10 % en poids de matière sèche, soit d'un colorant à une teneur comprise entre 0,2 et 5% en poids, soit d'un mélange de charge pigmentaire et de colorant.

Dans l'application selon l'invention à des couvercles, ledit revêtement a un grammage G compris entre 5 et 15 g/m² et de préférence un grammage allant de 8 à 12 g/m² et peut contenir une teneur pondérale en pigment T comprise entre 1 et 10%, le rapport G/T étant avantageusement au moins égal à 1.

Ainsi, pour obtenir une force colorante suffisante, il peut être nécessaire d'augmenter G et T simultanément, afin que le rapport G/T conserve une valeur suffisamment élevée. En effet, il a été observé qu'un rapport G/T trop bas augmente les risques d'endommagement.

La demanderesse, suite aux essais réalisés, et comme cela ressort des exemples, a observé l'influence du grammage du vernis : plus celui-ci est élevé, toutes choses égales par ailleurs, et plus les valeurs d'IRE obtenues sont élevées.

La limite supérieure de 15 g/m² est une limite pratique au-delà de laquelle le revêtement est trop onéreux, soit à cause du coût direct de la matière constituant ledit revêtement, soit à cause du coût de fabrication, l'obtention d'un revêtement à grammage élevé pouvant exiger plusieurs passages de la bande ou du format métallique sur la ligne d'enduction ou de vernissage.

Dans l'application selon l'invention à des couvercles (5), ledit revêtement extérieur coloré (41) peut comprendre un constituant thermoplastique, notamment du PVC. Ce peut être un vernis organosol-vinylique avec un grammage G allant de 5 à 15 g/m², ou bien est un vernis bicouche avec une sous-couche (411) colorée de vernis époxy-phénolique avec un grammage G de 2 à 7 g/m² et avec une couche externe (412) de vernis organosol-vinylique avec un grammage G de 3 à 8 g/m² et, de préférence, non colorée.

On peut aussi utiliser comme revêtement extérieur (41) un vernis époxy-vinylique avec un grammage G élevé, typiquement de 8 à 12 g/m².

Il a été observé aussi des interactions entre pigment et revêtement : un même revêtement ne sera pas obligatoirement " bon " ou " mauvais " pour toutes les matières colorantes, même si certains types de revêtements, ceux comprenant un constituant thermoplastique, sont plutôt favorables.

La méthode peut être également appliquée à une bande métallique (4) comprenant un revêtement externe (41) coloré ou non servant à fabriquer les couvercles colorés ou non (5), ledit revêtement (41) étant sélectionné selon la méthode selon l'invention et à l'aide du dispositif (1) selon l'invention.

L'objet de l'invention ne se limite pas toutefois à la sélection des emballages à symétrie axiale. En effet, il importe de distinguer le fait que le dispositif de l'invention nécessite que l'élément d'emballage ou de matériau testé ait une symétrie axiale, de façon à avoir une libre rotation axiale durant le test, conformément au moyen c) de la revendication 1, du fait que le dispositif selon l'invention peut être utilisé pour résoudre tous les problèmes d'endommagements de revêtements constatés lors de processus industriels, quelle que soit son origine ou quelle que soit la forme des emballages.

Simplement, la sélection d'un revêtement à l'aide dudit dispositif doit être faite sur un objet à tester ayant une symétrie axiale.

L'invention s'applique donc aussi à la mise au point d'un revêtement de bande métallique pour des boîtes ou des couvercles de forme ou tout autre type d'emballage.

L'invention peut en outre s'appliquer à la mise au point de tout revêtement de support ou substrat métallique dans des domaines autres que celui de l'emballage, notamment à la mise au point de tôles laquées pour former des éléments de décoration ou de protection, que ce soit, par exemple, dans le domaine du bâtiment ou des transports et de la carrosserie automobile.

### EXEMPLES DE REALISATION

### I - DISPOSITIF UTILISE SELON L'INVENTION

Le dispositif (1) selon l'invention qui a été mis au point et utilisé est représenté aux figures 9-1 à 9-40, et aux figures 10 à 12.

Ledit dispositif (1) comprend :
* une table vibrante (10) de marque RETSCH ®, type VIBRO ® à taux de vibration ou efficacité réglable de 0 à 100% par variation de l'amplitude de la vibration, la fréquence étant constante à 50 Hz,
* un tube de guidage (13) en PVC de 60 mm de hauteur, 60 mm de diamètre intérieur, de 6,2 mm d'épaisseur, fixé à ladite table,
* un outil d'endommagement (2) comprenant une couronne (20) munie de 15 dents (23) régulièrement espacées angulairement et de poids égal à 139g, ladite fraction, égale à la fraction de longueur de circonférence où se trouvent des dents, étant égale à 0,25, une tige axiale (21) de 36 mm de hauteur portant une masselotte (22), constituée de n = 3 rondelles d'acier (220), chacune de 41g, le poids total étant dudit outil (2) étant de 262g. Les figures 9-1 à 9-40 et 12 schématisent le montage dudit dispositif comme déjà mentionné précédemment.

Les figures 10 et 11 sont des représentations avec les dimensions exactes de ladite couronne (20) qui permettent de la fabriquer par usinage.

### II - PROCEDE UTILISE

Comme déjà mentionné, les paramètres suivants ont été retenus :
- taux de vibration ou efficacité de la table vibrante : 60 %
   Voir à la figure 16-1 l'influence du taux de vibration sur l'IRE.
- durée de chaque essai : 3 minutes
   Voir à la figure 16-2 l'influence de la durée du test sur l'IRE.
- nombre de rondelles d'acier : 3 / masse totale dudit outil (2) : 262 g
   Voir à la figure 16-3 l'influence de la masse de l'outil d'endommagement (2) sur l'IRE.

Les deux paramètres " taux de vibration " et " masse de l'outil (2) " constituent les deux composantes de ladite intensité relative à l'action dudit moyen d'endommagement.

### III - ELEMENTS D'EMBALLAGES TESTES : EBAUCHES DE COUVERCLES

Les ébauches de couvercle sont des ébauches de couvercles standards de boîtes boisson dits " 202 " de diamètre extérieur égal à 59,5 mm, fabriquées sur presse à ébauche standard à partir de bande de métal verni.

Pour obtenir la bande de métal verni, on a verni, en utilisant soit des vernis du commerce, soit des vernis spécialement formulés dans le cadre de essais effectués pour la présente invention, une bande en alliage d'aluminium 5182 à l'épaisseur 0,224 mm, au moins sur une face, sur ligne industrielle de vernissage ou sur ligne pilote de vernissage pour les essais de laboratoire, dans des conditions de cuisson standard, avec équivalence entre cuisson sur ligne industrielle et sur ligne pilote.

Les vernis pigmentés sont typiquement obtenus, de manière bien connue des fabricants d'encres et de peintures, par incorporation et dispersion d'un pigment sous forme de poudre dans un vernis liquide à l'aide d'un moyen de dispersion, typiquement un broyeur à 3 cylindres, appelé aussi " tricylindre ".

Par ailleurs, dans le but de trouver des formulations de vernis colorés présentant une valeur élevée d'IRE, de très nombreux essais utilisant le dispositif selon l'exemple, ont été réalisés pour trouver l'influence des différents paramètres qui, outre la nature du vernis, peuvent intervenir dans les formulations (épaisseur de vernis, charge pigmentaire des vernis, nature du pigment, ...) ou l'application du vernis, notamment les conditions de cuisson (temps, température).

Ainsi, la demanderesse a trouvé que le grammage du vernis est un facteur de premier ordre, un grammage élevé (typiquement au-delà de 8-10 g/m²) conduisant à de meilleures performances de résistance à l'endommagement. Mais l'invention peut permettre justement d'éviter d'avoir recours à des grammages trop élevés, donc des couches épaisses de vernis, ce qui constitue au moins une économie de matière.

Par ailleurs, il a été trouvé que la résistance à l'endommagement varie aussi avec la charge en matière colorante (colorant ou pigment) : dans le cas de couches épaisses de vernis, la résistance diminue lorsque la charge en matière colorante augmente.

Les paramètres qui définissent un revêtement (vernis pigmenté) sont donc :
- la nature du vernis et/ou la référence commerciale du vernis,
- la nature ou couleur du pigment et la teneur pondérale en pigment dans le vernis coloré (pourcentage de la charge pigmentaire dans le vernis coloré sec), quand ils sont connus, comme dans le cas des essais de laboratoire effectués par la demanderesse elle-même,
- le grammage en vernis coloré.

### IV - RESULTATS D'IRE EN FONCTION DU REVETEMENT

### A) ESSAIS DE CORRELATION SELON L'INVENTION

Dans un premier temps, on a établi, à partir d'ébauches de couvercles colorées ou non, une corrélation entre les mesures d'IRE effectuées sur ligne industrielle (I_{I}) et avec le dispositif de laboratoire selon l'invention (I_{L}) :

| N° Essai | Revêtement | | Pigment | Endommagement | |
|---|---|---|---|---|---|
| | Nature | Grammage | | IL | I_{I} |
| | | | | | |
| E1-INCOLORE | Epoxy | 4g/m² | non | 0,11 | 0,38 |
| Réf. HOLDEN X1132 SLE | | | | | |
| Vernis industriel | | | | | |
| E2-COLORE ORANGE | Epoxy | 4g/m² | oui | 0,07 | 0,26 |
| Réf. HOLDEN HE 1236 | | | | | |
| Vernis industriel | | | | | |

Ces deux points E1 et E2 permettent de tracer une droite (8) dite de corrélation ou d'étalonnage (voir figure 13), qui donne une indication de la relation entre les valeurs de I_{L} et de I_{I}. Cette droite (8), définie que par deux points E₁ et E₂, n'est donc pas une " vraie " droite de corrélation ou de régression au sens on l'entend en statistique entre un ensemble de valeurs d'IRE sur ligne industrielle et avec le dispositif selon l'invention.

On a complété cette corrélation par les essais suivants :

| N° Essai - Référence du fournisseur | Revêtement | | Pigment | Endommagement | |
|---|---|---|---|---|---|
| | Nature | Grammage | | IL | I_{I} |
| 1- DEXTER4804 A10/D3 | Epoxy | 5,6g/m² | non | 0 ,04 | 0,26 |
| Vernis industriel | | | | | |
| lbis-HOLDEN LDC 772/1 | Epoxy | 6g/m² | oui | 0,06 | 0,38 |
| Vernis industriel Orange (30%) | | | | | |
| 2- ESSAI F5-5 (*) | O-V | 5,8g/m² | oui | 0,065 | 0,40 |
| Bleu (7,5%) | | | | | |
| 3- HOBA GDA232 | Polyester | 8g/m² | oui | 0,1 | 0,40 |
| Bleu Pepsi | | | | | |
| Vernis industriel | | | | | |
| 4-HOBA GDA 270 | Polyester | 8g/m² | oui | 0,1 | 0,38 |
| Bleu | | | | | |
| Vernis industriel | | | | | |
| 5-RHENANIA SC-270-2111 | Epoxy | 8g/m² | oui | 0,15 | 0,46 |
| Orange | | | | | |
| Vernis d'essai | | | | | |

| | | | | | |
|---|---|---|---|---|---|
| (*) vernis de laboratoire ayant une teneur pondérale de 7,5% de pigment bleu dans le vernis sec. | | | | | |

Ces 6 essais complémentaires confirment ladite corrélation - compte tenu de l'écart-type sur les valeurs de I_{L} et I_{I} qui varie de 0,03 à 0,06 entre les valeurs basses et les valeurs moyennes d'IRE.

On a porté sur la figure 13 la droite de corrélation (8') calculée sur la base des 6 essais précédents et des deux essais E1 et E1. Son coefficient de corrélation r vaut 0,83, ce qui correspond à une corrélation très significative.

Sur la base de cette droite de corrélation (8'), on peut conclure qu'un indice d'IRE de 0,35 environ mesuré avec le dispositif de laboratoire conduit à un indice d'IRE de 1 sur ligne industrielle.

### B) SELECTION DE REVETEMENTS AU LABORATOIRE & ESSAIS INDUSTRIELS

Dans un second temps, on a testé avec le dispositif de laboratoire selon l'invention toute une série de vernis colorés ou non, où " O-V " désigne un vernis organosol-vinylique, et retenu les vernis suivants d'indice IL au moins égal à 0,15. Sur le tableau suivant ont été portées les valeurs correspondantes de I_{I} mesurées sur les couvercles testés sur ligne de production :

| N° Essai - Fournisseur | Revêtement | | Pigment | Endommagement | |
|---|---|---|---|---|---|
| | Nature | Grammage | | I_{L} | I_{I} |
| 6- ESSAI ROUGE F2-10 (*) | O-V | 10g/m² | oui | 0,15 | 0,72 |
| 7- ESSAI NOIR PRINTEX(*) | O-V | 10g/m² | oui | 0,17 | 0,72 |
| F4-10 | | | | | |
| 8- BICOUCHE ROUGE | | 8g/m² | oui | 0,21 | 0,58 |
| Couche interne 4805 A02D/1 | Epoxy | 4g/m² | oui (**) | | |
| Couche interne 4805 A02D/1 | Epoxy | 4g/m² | oui (**) | | |
| Couche externe 4800A04R | O-V | 4g/m² | non | | |
| Vernis DEXTER | | | | | |
| 9- BICOUCHE VERT | | 8g/m² | oui | 0,22 | 0,60 |
| Couche interne 4806 A 01D/1 | Epoxy | 4g/m² | oui (**) | | |
| Couche externe 4800A04R | O-V | 4g/m² | non | | |
| Vernis DEXTER | | | | | |
| 10- BICOUCHE BLEU PEPSI | | 8g/m² | oui | 0,23 | 0,80 |
| Couche interne 4808 A 17R | Epoxy | 4g/m² | oui (**) | | |
| Couche externe 8510 A06R | O-V | 4g/m² | non | | |
| Vernis DEXTER | | | | | |
| 11- ESSAI NOIR PRINTEX (*) | O-V | 10g/m² | oui | 0,23 | 0,76 |
| F2-10 | | | | | |
| 12- ESSAI BLEU F2-10(*) | O-V | 10g/m² | oui | 0,23 | 0,67 |
| 13- ESSAI ROUGE F5-10 (*) | O-V | 10g/m² | oui | 0,27 | 0,82 |
| 14- ESSAI JAUNE F2-10 (*) | O-V | 10g/m² | oui | 0,29 | 0,71 |
| 15-ESSAI BLEU F5-10 (*) | O-V | 10g/m² | oui | 0,30 | 0,82 |
| 16- ESSAI NOIR PRINTEX (*) | O-V | 10g/m² | oui | 0,32 | 0,85 |
| F5-10 | | | | | |
| 17- ESSAI JAUNE F5-10 (*) | O-V | 10g/m² | oui | 0,38 | 0,80 |
| 18- ESSAI NOIR CIBA (*) | O-V | 10g/m² | oui | 0,38 | 0,80 |
| F5-10 | | | | | |
| 19- DEXTER 8510 A 06R | O-V | 8g/m² | non | 0,30 | 1,00 |
| Essai / couleur OR | | | | | |
| 20- DEXTER 8510 A 06R | O-V | 10g/m² | non | 0,50 | 0 ,99 |
| Essai / couleur OR | | | | | |
| 21- DEXTER 8510 A 06R | O-V | 12g/m² | non | 0,57 | 0,98 |
| Essai / couleur OR | | | | | |

| | | | | | |
|---|---|---|---|---|---|
| (*) essais de laboratoire à base de vernis O-V DEXTER 8510 A 06R, la série F2 contenant 15% en poids de pigment dans le vernis sec, la série F4 contenant 30% en poids de pigment dans le vernis sec et la série F5 contenant 7,5% en poids de pigment dans le vernis sec. (**) dans ce cas, le pigment a été remplacé par un colorant. | | | | | |

Il pourrait être également avantageux d'utiliser l'essai 19, qui conduit à un I_{L} de 0,30, pour étalonner le dispositif et la méthode selon l'invention, en complément des points E1 et E2 situés à des valeurs plus basses d'IRE.

Comme déjà indiqué, les paramètres opératoires du dispositif de laboratoire ont été choisis de manière à être plus sévères au test de laboratoire pour avoir discrimination entre vernis dans un domaine où ils présentent déjà un certain niveau de performance, le but étant de distinguer les " meilleurs " vernis parmi les " bons ", et non pas les " mauvais " parmi les " pires". En conséquence, on observe un effet d'écrasement des valeurs élevées d'I_{I} (essais 20 et 21).

On a porté sur la figure 13 la nouvelle droite de corrélation (8") calculée sur l'ensemble des points du diagramme de la figure 13, à l'exception des points 20 et 21 qui ne sont pas significatifs compte tenu de l'effet d'écrasement de I_{I} pour ces points.

Le coefficient de corrélation r vaut 0,89, ce qui correspond, compte tenu du nombre de points, à une corrélation très significative. La corrélation serait sans doute encore meilleure si chaque résultat avait été remplacé par une moyenne de 4 résultats, compte tenu des écarts-type. Avec une seule mesure, pour I=0,33, à titre indicatif, on a I = 0,38 +/- 0,08, avec une probabilité de 95%.

En conclusion, cet ensemble de résultats valide d'une part la méthode proposée pour mesurer un indice de résistance à l'endommagement, et permet d'autre part, la méthode ayant été validée une fois pour toutes, de sélectionner rapidement et à peu de frais des vernis colorés ou non pour couvercles ou autres emballages, qui satisfont au niveau d'exigences souhaité en ce qui concerne la résistance à l'endommagement.

Il est à remarquer combien sont proches les trois droites de la figure 13, la droite (8) formée à partir de 2 points (= 2 essais), la droite (8') de régression formée à partir de 8 (2+6) points ou essais, et la droite (8") de régression formée à partir de 22 points ou essais (2+6+14).

Ces essais et ces résultats montrent :
- d'une part, l'influence du grammage du vernis : voir par exemple les essais 20 à 22.

Ce grammage doit être supérieur à 5g/m², et de préférence aller de 8 à 12 g/m². Au-delà de 12 g/m², les coûts de matière et de fabrication tendent à devenir trop importants, car le vernissage devra être réalisé en plusieurs passes compte tenu de l'épaisseur finale du vernis. De préférence, le grammage utilisé sera le grammage le plus élevé qui puisse être obtenu en une seule passe, ce grammage-limite pouvant varier selon la nature des vernis.
- d'autre part, l'influence de la nature du vernis, le vernis préféré étant un vernis organosol-vinylique, connu pour comprendre une part de matière thermoplastique.
- enfin, l'influence de la teneur pondérale T en pigment dans le vernis sec : plus elle est élevée, plus faible est la valeur de l'IRE, un vernis non pigmenté ayant un IRE supérieur à un vernis pigmenté. Par ailleurs, ces essais, ainsi que d'autres essais complémentaires, ont montré qu'il était avantageux d'avoir toujours G > T, G étant le grammage du vernis coloré.

De préférence, les couvercles colorés selon l'invention comprennent la combinaison de moyens suivante : a) le revêtement est à base de vernis organosol-vinylique (O-V) ou comprend une couche externe non colorée en O-V, la couche intérieure étant en époxy colorée, b) le grammage G va de 8 à 12 g/m², et c) le rapport G/T est d'au moins 1.

### V - ESSAIS DE CORRELATION SELON L'ETAT DE LA TECHNIQUE

Ces essais ont été réalisés, à l'aide de méthodes de caractérisation ou de contrôle connues, sur des échantillons de métal verni pigmenté ou non ayant servi à fabriquer les ébauches testées précédemment selon l'invention.

Comme illustré sur les figures 3 à 5, il n'y a pas de corrélation entre les valeurs d'IRE mesurées à l'issue dudit processus industriel pour différents revêtements testés, et les trois types de caractérisation initialement essayées par la demanderesse :

En ce qui concerne le test de la dureté au crayon (voir figure 3), on voit que les 4 vernis testés ont la même dureté au crayon, alors que les valeurs d'IRE varient du simple au triple.

En ce qui concerne le test Taber ® (voir figure 4), on voit qu'il n'y a pas de corrélation entre les valeurs d'IRE et la perte de poids pour les 5 vernis testés, puisqu'on trouve, par exemple, pratiquement une même valeur d'IRE pour les valeurs extrêmes de perte de poids. Les test ont été réalisés avec des roues abrasives CS 10 comme cité dans la norme ASTM D 4060.

En ce qui concerne le test de micro-rayure (voir figure 5), peut-être y a-t-il dans ce cas une faible corrélation entre la distance D_{A} et l'IRE, mais cette corrélation - si corrélation il y a - est trop faible pour présenter un quelconque intérêt prédictif de la résistance à l'endommagement d'un revêtement donné.

La demanderesse a aussi essayé - sans plus de succès - la méthode dite de quadrillage, selon la norme ISO 2409 ou ASTM D 3359, dans laquelle, à partir d'un échantillon de métal verni, on réalise une grille d'incision (traits parallèles incisés), on applique un ruban adhésif, et, après avoir retiré le ruban adhésif, on examine la quantité de vernis entraîné par ledit ruban adhésif.

### VI - CONCLUSION GENERALE

D'une part, la demanderesse a mis au point, compte tenu de la non efficacité des moyens connus, un dispositif et une méthode de mesure d'IRE, et a montré que les résultats obtenus au laboratoire, par un test rapide et peu coûteux, étaient corrélés aux résultats obtenus sur lignes industrielles.

D'autre part, la demanderesse a trouvé les conditions expérimentales qui permettent d'obtenir des valeurs élevées d'IRE dans le cas des couvercles pour boîtes boisson.

Ainsi, le dispositif et la méthode proposée pour mesurer un IRE permet de sélectionner, rapidement et à peu de frais, des vernis colorés ou non pour couvercles ou autres emballages, ou tout autre support métallique revêtu ou verni, qui satisfont au niveau d'exigences souhaité en ce qui concerne la résistance à l'endommagement.

### AVANTAGES DE L'NVENTION

L'invention constitue un enseignement technique précieux, notamment pour le fabricant d'emballages métalliques mais aussi dans d'autres domaines ou métiers où se posent les mêmes problèmes d'endommagement (automobile, bâtiment...), dans la mesure où, à l'aide d'un dispositif de laboratoire peu coûteux, il permet d'étudier et de sélectionner, rapidement et à peu de frais, comme cela a été montré dans le cas de la recherche de couvercles colorés, les revêtements les plus aptes à répondre à des contraintes imposées par l'ensemble des processus industriels, tout au long de la chaîne allant des matériaux de départ jusqu'au client final.

Le dispositif selon l'invention, ainsi que la méthode, peuvent être ainsi utilisés et adaptés à l'examen de tout support métallique recouvert d'un revêtement susceptible d'être mécaniquement endommagé, qu'il s'agisse par exemple d'ustensiles en métal laqué, panneaux de bardage dans la construction de bàtiments, ou de carrosserie automobile.

En effet, les chocs d'origine mécanique peuvent avoir de très nombreuses origines, à partir du moment où, par exemple, ledit emballage n'est pas strictement immobile par rapport à son environnement immédiat. Pour que ces chocs, en grande partie inévitables, ne donnent pas lieu à endommagement, le fabricant de supports métalliques revêtus, et en particulier le fabricant d'emballages, ne peut qu'agir sur le revêtement, d'où le grand intérêt pratique de la présente invention.

Par ailleurs, la présente invention divulgue une combinaison de moyens (nature du vernis, grammage du vernis, rapport G/T) qui ne s'appliquent pas seulement au cas des couvercles colorés, mais sans aucun doute à toutes sortes de cas, dans le domaine ou non de l'emballage, où des problèmes similaires d'endommagement par chocs mécaniques se posent.

## Revendications

1. Dispositif de laboratoire (1) pour caractériser la résistance à l'endommagement d'un revêtement protecteur (41), film plastique ou vernis, de tout ou partie d'un emballage, d'un substrat ou d'un élément d'emballage métallique (3,5,30,31,32,33), comprenant une couche métallique (42), destiné à reproduire l'endommagement résultant de processus industriels donnés, typiquement dus à l'empilement ou au gerbage desdits emballages conduisant à des plages de contact (52,312,301,302,313,314,321,322, 323,313) sous forme de pistes typiquement circulaires dans le cas d'emballages à bords circulaires, et permettant de mesurer un indice de résistance à l'endommagement - IRE en abrégé - typiquement par un ratio de "zone non endommagée / zone totale considérée ", comprenant un moyen d'endommagement (2) soumis à un mouvement vibratoire et des moyens de régulation de l'action dudit moyen d'endommagement, typiquement en intensité, de manière à pouvoir notamment étalonner ledit dispositif par rapport audit processus industriel, ledit dispositif étant_**caractérisé en ce que** ledit moyen d'endommagement (2) comprend une couronne (20), munie de dents (23), de dureté élevée supérieure à celle de ladite couche métallique, typiquement en carbure de tungstène, et **en ce que** ledit dispositif comprend en outre :
a) des moyens vibratoires assurant une succession de chocs de ladite couronne, dans le sens vertical, contre ladite piste (52),
b) des moyens assurant que, lors de chaque choc, ladite couronne ne s'applique simultanément que sur une fraction de ladite piste circulaire dudit emballage
c) des moyens assurant la libre rotation relative de ladite couronne de telle manière que ladite couronne s'applique aléatoirement sur toute partie de ladite piste.

2. Dispositif selon la revendication 1 dans lequel ledit moyen (2) et ladite piste (52) sont, chacun, libres en rotation axiale.

3. Dispositif selon la revendication 2 comprenant :
a) une table vibrante (10), à taux de vibration ou efficacité modulable, typiquement en amplitude, assurant ledit mouvement vibratoire,
b) un tube de guidage vertical (13) fixé à ladite table vibrante (10), dans lequel peut glisser ou tourner autour d'un axe vertical, sans frottement notable, ledit emballage ou élément d'emballage métallique, et, au-dessus de celui-ci, ledit moyen ou outil d'endommagement (2),
c) une tige axiale (21) surmontant ladite couronne et portant une masselotte (22) formée d'un empilement d'un nombre de rondelles d'acier (220) choisi en fonction de ladite intensité, ledit outil (2) étant placé dans ledit tube de guidage (13).

4. Méthode de mesure d'un indice de résistance à l'endommagement - IRE en abrégé - typiquement par un ratio de " zone non endommagée / zone totale considérée ", avec le dispositif (1) selon une quelconque des revendications 1 à 3, et corrélé à l'endommagement résultant d'un processus industriel, dans laquelle :
a) on approvisionne au moins un type d'emballage ou d'élément d'emballage donné Eₒ et on le soumet audit processus industriel, de manière à obtenir un emballage E_{I} dont le revêtement a subi un certain endommagement à l'issue dudit processus industriel, et sur lequel on mesure une valeur I_{I} pour l'IRE,
b) on soumet ledit emballage ou élément d'emballage Eₒ, comprenant ladite plage de contact, audit dispositif, durant une durée et avec ladite intensité choisies de façon à obtenir un emballage modifié en laboratoire E_{L}, présentant un endommagement dont la valeur IL mesurée est typiquement voisine de 0,3.I_{I}, et ainsi à fixer, une fois pour toutes pour un processus industriel donné, des conditions opératoires dudit dispositif et de ladite méthode, pour obtenir un niveau d'endommagement de préférence supérieur à celui obtenu lors dudit processus industriel, et ainsi de pouvoir évaluer la résistance à l'endommagement desdits revêtements (41) étudiés, classer les revêtements par IRE et sélectionner au moindre coût et rapidement les revêtements les plus performants.

5. Méthode selon la revendication 4 dans laquelle :
a) on approvisionne deux types d'emballages E₁ et E₂, conduisant à l'issue dudit processus industriel, à des valeurs distinctes, et de préférence éloignées l'une de l'autre, I_{I1} et I_{I2} pour l'IRE, l'emballage E₁ étant revêtu d'un premier vernis de référence et l'emballage E₂ étant revêtu d'un second vernis de référence,
b) on soumet un des emballages, par exemple E₂, audit dispositif (1) de laboratoire et on règle ladite durée et intensité, de manière à obtenir une valeur d'endommagement correspondante I_{L2} telle que I_{L2} soit typiquement de l'ordre de 0,3.I_{I2},
c) les réglages de durée et intensité étant déterminés et fixés en b), on soumet l'emballage E₁ ou tout autre emballage à étudier, audit dispositif et on mesure la valeur I_{L1} correspondante,
d) à partir des deux couples de valeurs I_{L1} et I_{I1}, I_{L2} et I_{I2}, on obtient un droite d'étalonnage, dite de corrélation (8), entre les valeurs d'endommagement des revêtements (41) mesurées sur emballages à l'issue d'un processus industriel et à l'issue de l'endommagement avec ledit dispositif (1), de façon à pouvoir, compte tenu d'une valeur limite I_{I-lim} reconnue comme acceptable pour ledit emballage à l'issue dudit processus industriel, calculer une valeur correspondante théorique I_{L-lim} ainsi qu'une valeur pratique compte tenu des intervalles de confiance associés à une seule mesure avec ledit dispositif, et sélectionner rapidement et au moindre coût lesdits revêtements (41) susceptibles de convenir à l'échelle industrielle.

6. Méthode selon une quelconque des revendications 4 et 5 dans laquelle les IRE sont mesurés soit par un rapport de longueur de piste ou couronne non endommagée (521) sur la longueur totale de piste ou de couronne, soit par un rapport de surface non endommagée sur surface totale considérée, une partie endommagée étant une partie d'emballage où ladite couche métallique a été mise à nu suite audit endommagement, ladite longueur totale de piste ou de couronne correspondant typiquement au rebord circulaire d'un corps de boîte (30,32,33), d'un couvercle (5), ou d'une boîte sertie (32,33).

7. Méthode selon une quelconque des revendications 4 à 6 dans laquelle :
- ledit emballage Eₒ est une ébauche (31) de couvercle rond de boîte boisson de 59,5 mm de diamètre revêtu extérieurement d'un vernis époxy-phénolique à 4 g/m² constituant ledit premier vernis de référence,
- ledit dispositif (1) comprend :
* une table vibrante (10) fonctionnant à 50 Hz et à amplitude variable,
* un tube de guidage (13) en PVC de 60 mm de hauteur, 60 mm de diamètre intérieur, de 6,2 mm d'épaisseur,
* un outil d'endommagement (2) comprenant une couronne (20) munie de 15 dents (23) régulièrement espacées angulairement et de poids égal à 139 g, ladite fraction, égale à fraction de longueur de circonférence où se trouvent des dents, étant égale à 25%, une tige axiale (21) de 36 mm de hauteur portant une masselotte (22), constituée de n rondelles d'acier (220), chacune de poids unitaire égal à 41 g,
- on prend le nombre n égal à 3, le poids dudit outil étant alors de 262g, on fixe ladite durée à 3 min, et on règle le taux de vibration ou d'efficacité de ladite table vibrante à 60%, ces paramètres opératoires étant fixés pour obtenir une valeur IL de 0,11 de l'ordre de 0,3.I_{I}, de manière à ce que l'endommagement avec le dispositif de laboratoire soit plus sévère que l'endommagement lors du processus industriel et que la transposition d'une sélection desdits revêtements (41), du laboratoire au processus industriel, soit d'autant plus fiable, ledit processus étant typiquement le convoyage des ébauches (31) de couvercles sur toute la ligne de production de couvercles qui conduit typiquement à une valeur I_{I} de 0,38.

8. Méthode selon les revendications 5 et 7 dans laquelle :
- lesdits emballages E₁ et E₂ sont des ébauches (31) de couvercles de boîte boisson, l'emballage E₁ étant revêtu extérieurement d'un vernis époxy-phénolique à 4 g/m² constituant ledit premier vernis de référence, l'emballage E₂ étant revêtu extérieurement d'un vernis de même nature et à 4g/m², pigmenté orange, constituant ledit second vernis de référence,
- les mêmes dits paramètres opératoires étant conservés, la valeur d'IRE I_{L2} relative à l'emballage E₂ est déterminée avec ledit dispositif et trouvée égale à 0,045,
- on mesure les valeurs correspondantes de I_{I1} et I_{I2} sur lesdits emballages E₁ et E₂ issus dudit processus industriel, valeurs trouvées respectivement égales à 0,38 et 0,26,
- ladite valeur limite I_{I-lim} ayant été estimée à 0,60, on détermine, sur la base de ladite droite d'étalonnage ou de corrélation (8), ladite valeur limite théorique E_{L-lim} correspondante de 0,23 et une valeur E_{L-lim} pratique de 0,15 compte tenu des intervalles de confiance.

9. Méthode selon une quelconque des revendications 4 à 8 dans laquelle ledit emballage Eo, ou lesdits emballages E₁ et E₂ sont remplacés, en ce qui concerne l'utilisation dudit dispositif (1) de laboratoire, par les matériaux de départ correspondants, ledit moyen d'endommagement étant dès lors appliqué non pas sur un emballage, mais sur le revêtement (41) de ladite bande métallique (4) destinée à former ledit emballage métallique.

10. Utilisation du dispositif selon une quelconque des revendications 1 à 3 pour la caractérisation de tout revêtement de support métallique dans des domaines autres que celui de l'emballage, notamment pour la mise au point de tôles laquées pour former des éléments de décoration ou de protection, que ce soit, par exemple, dans le domaine du bâtiment ou des transports et de la carrosserie automobile.

## Claims

1. A laboratory device (1) for characterizing the resistance to damage of a protective surface (41), plastic film or varnish, of the whole or a part of packaging, of a substrate or element of metallic packaging (3,5,30,31,32,33), consisting of a metallic layer (42), intended to reproduce the damaging resulting from given industrial processes, typically due to piling up or stacking said packaging leading to contact zones (52,312,301,302,313,314,321,322, 323) in the form of typically circular tracks in the case of packaging with circular edges, and making it possible to measure a damage resistance index - DRI in abbreviated form - typically by a ratio of the undamaged zone to the total zone under consideration, including a damaging device (2) subjected to a vibratory movement and of a means of regulating the action of said damaging device, typically in intensity, so as to be able to notably calibrate said device in relation to said industrial process, said device being **characterized by** the fact that said damaging device (2) consists of a crown (20), provided with teeth (23), of hardness greater than that of said metallic layer, typically made of tungsten carbide, and in that said device also comprises:
a) vibratory means, sending a succession of impacts from said crown, in a vertical direction, against said track (52),
b) means ensuring that, at each impact, said damaging device is applied simultaneously to only a fraction of said circular track of said packaging
c) means assuring that said crown is free to rotate so that said crown is applied randomly to any part of said track.

2. Device as recited in claim 1 wherein said means (2) and said track (52) are both free to rotate axially.

3. Device as recited in claim 2 comprising:
a) a vibrating table (10), with an adjustable rate of vibration or efficiency, typically in wavelength, providing said vibratory movement,
b) a vertical guide tube (13) fixed to said vibrating table (10), in which said metallic packaging or packaging element can slide or rotate around a vertical axis, without noticeable friction, and above this, said damaging device or tool (2),
c) an axial rod (21) topping said crown and bearing a weight (22) formed by a stack of a number of steel washers (220) chosen according to said intensity, said tool (2) being placed in said guide tube (13).

4. A measurement method to give a damage resistance index - DRI in abbreviated form - typically by a ratio of the undamaged zone to the total zone under consideration, with the device (1) according to any of claims 1 - 3, and correlated with the damage resulting from an industrial process, in which:
a) at least one given type of packaging or packaging element E₀ is procured and is subjected to said industrial process, so as to obtain a packaging E_{I} the coating of which has undergone certain damage following said industrial process, and on which a value I₁ for the DRI is measured,
b) said packaging or packaging element E₀, comprising said contact zone, is subjected to said device for a chosen period of time and with said intensity chosen so as to obtain an in-laboratory modified packaging E_{L}, with damage of which the measured value I_{L} is typically close to 0.3 I_{I}. and thereby to fix, once and for all for a given industrial process, operating conditions for said device and said method, to obtain a damage level, preferably greater than that obtained during said industrial process, and thereby to be able to assess the damage resistance of said surfaces (41) examined, rank surfaces by DRI and select quickly and at low cost the most efficient coatings.

5. A method as recited in claim 4 wherein:
a) two types of packaging, E₁ and E₂, are procured, giving, at the end of the industrial process, distinct values, preferably distant from one another, I₁₁ and I₁₂ for the DRI, packaging E₁ being coated with a first reference varnish and E₂, being coated with a second reference varnish,
b) one of these packagings, for example E₂, is subjected to said laboratory device (1) and said time and intensity are adjusted so as to obtain a corresponding damage value I_{L2} such that I_{L2} is roughly 0.3 I₁₂,
c) the time and intensity settings being determined and fixed at b), packaging E₁ or any other packaging to be studied, is subjected to said device and the corresponding I_{L1} value is measured,
d) from the two pairs of values I_{L1} and .I₁₁, I_{L2} and I₁₂, a calibration line, known as a correlation line (8), is obtained between the coating damage values (41) measured on packaging at the end of the industrial process and at the end of the damaging process with said device (1), in order to be able, given a boundary value I_{I-LIM} acknowledged as acceptable for said packaging at the end of the industrial process, to calculate a corresponding theoretical value I_{L-LIM} as well as a practical value given the confidence intervals related to a single measure with said device, and to select quickly and at low cost said coatings (41) must likely to be suitable on an industrial scale.

6. A method as recited in any one of claims 4 and 5 wherein the DRIs are measured either by the ratio of the length of the undamaged track or crown (521) to the total length of the track or crown, or by the ratio of the undamaged surface area to the total surface area under consideration, a damaged part being a part of the packaging where said metallic layer has been laid bare as a result of said damage, said total length of the track or crown typically corresponding to the circular edge of a can body (30,32,33), a lid (5), or a crimped can (32,33).

7. A method as recited in any one of claims 4 to 6 wherein:
- said packaging E₀ is a round beverage can top blank (31) with an outside diameter of 59.5 mm, coated on the outside with an epoxy-phenolic varnish at 4 g/m² forming the first reference varnish.
- said device (1) comprises:
* a vibrating table (10) operating at 50 Hz and of variable wavelength,
* a PVC guide tube (13), 60 mm high, 60 mm inside diameter and 6.2 mm thick,
* a damage tool (2) comprising a crown (20) provided with 15 teeth (23) regularly spaced out and weighing 139 g., said fraction, equal to the fraction of the length of the circumference where the teeth are, being 25%, an axial rod (21) 36 mm high bearing a weight (22), made up of n steel washers (220) each weighing 41 g,
- n is taken to be equal to 3, the total weight of said tool being then 262g, said time is fixed at 3 min., and the rate of vibration or efficiency of said vibrating table at 60%, these operating parameters being fixed to obtain a value for I_{L} of 0.11 or roughly 0.3 I₁ so that the damage caused by the laboratory device is greater than the damage caused as a result of the industrial process and that the transposition of said coatings (41) from the laboratory to the industrial process is all the more reliable, said process being typically the conveying of can top blanks (31) over the entire can top production line, which typically gives a value for of 0.38.

8. A method as recited in claims 5 and 7 wherein:
- said packagings E₁ and E₂ are beverage can top blanks (31), packaging E₁ being coated on the outside with an epoxy-phenolic varnish at a rate of 4g/m² forming the first reference varnish, packaging E₂ being coated on the outside with varnish of the same kind and at a rate of 4g/m², colored with orange pigment, forming the second reference varnish,
- the same said operating parameters being conserved, the DRI value I_{L2} relative to packaging E2 is determined with said device and found to be 0.045,
- the corresponding values of I₁₁ and I₁₂ are measured, on said packagings E₁ and E₂ from said industrial process; these values are found to be 0.38 and 0.26 respectively,
- said boundary value I_{L-lim} having been estimated at 0.60, said corresponding theoretical boundary value E_{L-LIM} of 0.23 is determined on the basis of said calibration or correlation line (8), and a practical value of 0.15 given the confidence intervals is also determined.

9. A method as recited in any of claims 4 to 8 in which said packaging Eo, or said packagings E and E are replaced, from the standpoint of using said laboratory device (1), by corresponding basic materials, said damaging device being then applied not to a packaging but the coating (41) of said metallic strip (4) intended to form said metallic packaging.

10. Use of the device as recited in any one of claims 1 to 3 to characterize any coating of a metallic medium in fields other than those of packaging, notably for the development of lacquered sheet metal to form decorative or protective elements, for example in the building or transport industry and for automobile bodywork.

## Patentansprüche

1. Laborvorrichtung (1) zur Kennzeichnung der Beschädigungsfestigkeit einer Schutzbeschichtung (41), Plastikfilm oder Lack, einer ganzen Metallverpackung oder eines Teils davon, eines Metallsubstrats oder eines Metallverpackungselementes (3, 5, 30, 31, 32, 33) mit einer Metallschicht, welche Vorrichtung dazu bestimmt ist, die Beschädigung aus gegebenen Industrieprozessen, typischerweise durch Stapeln oder Aufeinanderschichten der Verpackungen, was bei Verpackungen mit kreisförmigen Rändern zu Kontaktbereichen (53, 312, 301, 302, 313, 314, 321, 322, 323, 313) in Form von typischerweise kreisförmigen Bahnen führt, zu reproduzieren und es ermöglicht, einen Beschädigungsfestigkeitsindex zu messen, typischerweise durch ein Verhältnis von "nicht beschädigtem Bereich" zu "betrachtetem Gesamtbereich", umfassend ein Beschädigungsmittel (2), das einer Vibrationsbewegung ausgesetzt wird, und Mittel zur Regulierung der Tätigkeit des Beschädigungsmittels, typischerweise hinsichtlich der Intensität, um die Vorrichtung insbesondere in Bezug auf den Industrieprozess kalibrieren zu können, wobei die Vorrichtung **dadurch gekennzeichnet ist, dass** das Beschädigungsmittel (2) einen mit Zähnen (23) versehenen Kranz (20) aufweist, dessen hohe Härte höher ist als die der Metallschicht und der typischerweise aus Wolframkarbid besteht, und dass die Vorrichtung weiterhin umfasst:
a) Vibrationsmittel, die in vertikaler Richtung eine Folge von Stößen des Kranzes gegen die Bahn (52) bewirken,
b) Mittel, die bewirken, dass der Kranz bei jedem Stoß gleichzeitig nur auf einen Bruchteil der kreisförmigen Bahn der Verpackung aufgebracht wird,
c) Mittel, die eine relative Freidrehung des Kranzes bewirken, derart, dass der Kranz aleatorisch auf alle Teile der Bahn aufgebracht wird.

2. Vorrichtung nach Anspruch 1, bei der das Mittel (2) und die Bahn (52) jeweils axial frei drehbar sind.

3. Vorrichtung nach Anspruch 2, umfassend:
a) einen die Vibrationsbewegung bewirkenden Vibriertisch (10) mit modulierbarem Vibrations- oder Leistungsgrad, typischerweise hinsichtlich der Amplitude,
b) ein am Vibriertisch (10) befestigtes vertikales Führungsrohr (13), in dem die Metallverpackung oder das Metallverpackungselement ohne nennenswerte Reibung gleiten oder sich um eine vertikale Achse drehen kann, und über diesem das genannte Beschädigungsmittel oder -werkzeug (2),
c) eine den Kranz überragende Axialstange (21), welche ein Gewicht (22) trägt, das aus einer je nach der Intensität gewählten Anzahl von aufgestapelten Stahlscheiben (220) besteht, wobei das Werkzeug (2) in dem Führungsrohr (13) angeordnet ist.

4. Verfahren zur Messung eines Beschädigungsfestigkeitsindex, typischerweise durch ein Verhältnis von "nicht beschädigtem Bereich" zu "betrachtetem Gesamtbereich", mit der Vorrichtung (1) nach irgendeinem der Ansprüche 1 bis 3 und in Korrelation mit der Beschädigung aus einem Industrieprozess, bei dem:
a) mindestens ein gegebener Verpackungs- oder Verpackungselementtyp Eₒ bereitgestellt und dem Industrieprozess unterworfen wird, um eine Verpackung E_{I} zu erhalten, deren Beschichtung nach dem Industrieprozess eine gewisse Beschädigung erfahren hat und an der ein Wert I_{I} für den Beschädigungsfestigkeitsindex gemessen wird,
b) die Verpackung oder das Verpackungselement Eₒ mitsamt dem genannten Kontaktbereich der Vorrichtung unterworfen wird, für eine Dauer und mit der genannten Intensität, die so gewählt werden, dass eine laborseitig modifizierte Verpackung E_{L} mit einer Beschädigung entsteht, deren gemessener Wert I_{L} typischerweise etwa 0,3.I_{I} beträgt, und auf diese Weise ein für alle Mal für einen gegebenen Industrieprozess Durchführungsbedingungen für die Vorrichtung und das Verfahren festgelegt werden, um ein vorzugsweise höheres Beschädigungsniveau als das beim Industrieprozess erhaltene zu gewinnen und so die Beschädigungsfestigkeit der untersuchten Beschichtungen (41) bewerten, die Beschichtungen nach dem Beschädigungsfestigkeitsindex ordnen und die wirksamsten Beschichtungen mit geringsten Kosten schnell auswählen zu können.

5. Verfahren nach Anspruch 4, bei dem:
a) zwei Verpackungstypen E₁ und E₂ bereitgestellt werden, die nach dem Industrieprozess zu verschiedenen, vorzugsweise voneinander entfernten Werten I_{I1} und I_{I2} für den Beschädigungsfestigkeitsindex führen, wobei die Verpackung E₁ mit einem ersten Referenzlack und die Verpackung E₂ mit einem zweiten Referenzlack beschichtet ist,
b) eine der Verpackungen, zum Beispiel E₂, der Laborvorrichtung (1) unterworfen wird und die Dauer und Intensität so eingestellt werden, dass sich ein einsprechender Beschädigungswert I_{L2} ergibt und I_{L2} typischerweise etwa 0,3.I_{I2} beträgt,
c) wenn die Einstellungen hinsichtlich Dauer und Intensität in b) bestimmt und festgelegt sind, die Verpackung E₁ oder eine beliebige andere zu untersuchende Verpackung der Vorrichtung unterworfen und der entsprechende Wert I_{L1} gemessen wird,
d) aus den zwei Wertepaaren I_{L1} und I_{I1}, I_{L2} und I_{I2} eine Kalibrier- oder sog. Korrelationsgerade (8) zwischen den an den Verpackungen nach einem Industrieprozess und nach der Beschädigung gemessenen Beschädigungswerten der Beschichtungen (41) mit der Vorrichtung (1) gewonnen wird, um unter Berücksichtigung eines für die Verpackung nach dem Industrieprozess als akzeptabel angesehenen Grenzwertes I_{I-lim} einen entsprechenden theoretischen Wert I_{L-lim} sowie unter Berücksichtung der mit einer einzigen Messung mit der Vorrichtung verbundenen Vertrauensintervalle einen praktischen Wert berechnen zu können und Beschichtungen, die sich für einen großtechnischen Einsatz eignen, mit geringsten Kosten schnell auswählen zu können.

6. Verfahren nach irgendeinem der Ansprüche 4 und 5, bei dem die Beschädigungsfestigkeitsindexe entweder durch ein Verhältnis von nicht beschädigter Bahn- oder Kranzlänge (521) zu Bahn- oder Kranzgesamtlänge oder durch ein Verhältnis von nicht beschädigter Oberfläche zu betrachteter Gesamtoberfläche gemessen werden, wobei ein beschädigter Bereich ein Verpackungsbereich ist, wo die Metallschicht aufgrund der Beschädigung bloßgelegt wurde, und die Bahn- oder Kranzgesamtlänge typischerweise dem kreisförmigen Rand eines Dosenkörpers (30, 32, 33), eines Deckels (5) oder einer verschlossenen Dose (32, 33) entspricht.

7. Verfahren nach irgendeinem der Ansprüche 4 bis 6, bei dem:
- die Verpackung Eₒ ein Vormaterial (31) für einen runden Getränkedosendeckel von 59,5 mm Durchmesser ist, welcher außen mit 4 g/m² Epoxyphenollack beschichtet ist, der den ersten Referenzlack darstellt,
- die Vorrichtung (1) umfasst:
* einen Vibriertisch (10), der mit 50 Hz und variierbarer Amplitude arbeitet,
* ein Führungsrohr (13) aus PVC von 60 mm Höhe, 60 mm Innendurchmesser, 6,2 mm Dicke,
* ein Beschädigungswerkzeug (2) bestehend aus einem Kranz (20) mit 15 regelmäßig im Winkel voneinander beabstandeten Zähnen (23) mit einem Gewicht von 139 g, wobei der Abschnitt, der dem Umfangslängenabschnitt mit den Zähnen entspricht, 25 % beträgt, und einer 36 mm hohen Axialstange (21), die ein Gewicht (22) trägt, welches aus n Stahlscheiben (220) mit einem jeweiligen Einzelgewicht von 41 g besteht,
- die Zahl n gleich 3 genommen wird, wobei das Gewicht des Werkzeugs dann 262 g beträgt, die Dauer auf 3 min festgelegt und der Vibrations- oder Leistungsgrad des Vibriertisches auf 60 % eingestellt wird, wobei diese Durchführungsparameter zur Gewinnung eines Wertes I_{L} von 0,11 in der Größenordnung von 0,3.I_{I} festgelegt werden, damit die Beschädigung mit der Laborvorrichtung stärker ist als die Beschädigung beim Industrieprozess und die Umsetzung einer Auswahl der Beschichtungen (41) vom Labor auf den Industrieprozess um so zuverlässiger ist, wobei es sich bei dem Industrieprozess typischerweise um die Beförderung der Vormaterialien (31) für Deckel über die gesamte Deckel-Produktionsstraße handelt, die typischerweise zu einem Wert I_{I} von 0,38 führt.

8. Verfahren nach den Ansprüchen 5 und 7, bei dem:
- die Verpackungen E₁ und E₂ Vormaterialien (31) für Getränkedosendeckel sind, wobei die Verpackung E₁ außen mit einem 4g/m² Epoxyphenollack beschichtet wird, der den ersten Referenzlack darstellt, die Verpackung E₂ außen mit einem orange pigmentierten 4g/m² Lack gleicher Art beschichtet wird, der den zweiten Referenzlack darstellt,
- aufgrund dessen, dass die gleichen Durchführungsparameter beibehalten werden, der Wert des Beschädigungsfestigkeitsindex I_{L2} bezüglich der Verpackung E₂ mit der Vorrichtung bestimmt wird und gleich 0,045 ist,
- die entsprechenden Werte von I_{I1} und I_{I2} an den Verpackungen E₁ und E₂ aus dem Industrieprozess gemessen werden, welche Werte gleich 0,38 bzw. 0,26 sind,
- aufgrund dessen, dass der Grenzwert I_{I-lim} auf 0,60 geschätzt wurde, auf der Basis der Kalibrier- oder Korrelationsgeraden (8) der entsprechende theoretische Grenzwert E_{L-lim} von 0,23 und ein praktischer Wert E_{L-lim} von 0,15 angesichts der Vertrauensintervalle bestimmt wird.

9. Verfahren nach irgendeinem der Ansprüche 4 bis 8, bei dem die Verpackung Eₒ oder die Verpackungen E₁ und E₂ im Hinblick auf die Verwendung der Laborvorrichtung (1) durch die entsprechenden Ausgangswerkstoffe ersetzt werden, wobei das Beschädigungsmittel dann nicht auf eine Verpackung aufgebracht wird, sondern auf die Beschichtung (41) des Metallbandes (4) zur Herstellung der Metallverpackung.

10. Verwendung der Vorrichtung nach irgendeinem der Ansprüche 1 bis 3 für die Kennzeichnung beliebiger Beschichtungen von metallischem Trägermaterial in anderen Bereichen als dem Verpackungsbereich, insbesondere für die Entwicklung lackierter Bleche zur Herstellung von Dekorations- oder Schutzelementen, sei es zum Beispiel im Bereich des Bau- oder Transportwesens oder der Fahrzeugkarosserie.
